# DEMANDE DE BREVET EUROPEEN

(11) **EP 2 476 307 A2**
(43) Date de publication de la demande: **18.07.2012**
(21) Numéro de dépôt: 12151241.2
(22) Date de dépôt: 05.03.2008
(51) Int. Cl.: A01H 1/02, A01H 5/10

(54) **Production de graines lactuca sativa hybrides**

(30) Priorité: 05.03.2007 FR 0701589
(62) Demande divisionnaire de: 08775629.2
(71) Demandeur: Vilmorin, 49250 La Menitre (FR)
(72) Inventeur: Michel, Hervé, 49000 Angers (FR); Soussin, Thierry, 49630 Maze (FR)
(74) Mandataire: Almond-Martin, Carol

(57) **Abrégé**

La présente invention se rapporte à des graines de *Lactuca sativa* hybrides, présentant un génotype mâle stérile, et hétérozygotes pour au moins un gène distinct conférant un phénotype détectable aux plantes issues de ces graines. L'invention se rapporte également à un procédé de production de graines *Lactuca sativa* hybrides, comprenant une étape de culture dans un milieu fermé, de plantes phénotypiquement mâles stériles et de plantes phénotypiquement mâles fertiles, une étape de pollinisation par des diptères introduits dans le milieu fermé à l'époque de la floraison des plantes et une étape de récolte des graines produites par les plantes mâles stériles.

## Description

La présente invention se rapporte à des graines de laitues *(Lactuca sativa* hybrides, ainsi qu'à un procédé de production desdites graines. L'invention se rapporte également à des plantes *Lactuca sativa* hybrides, ainsi qu'à des cellules de plantes *Lactuca sativa* hybrides.

Au nombre des raisons de produire des graines de végétaux hybrides à grande échelle, on compte notamment l'hétérosis, (ou vigueur hybride), l'homéostasie (stabilité de la plante dans différents environnements), la possibilité de cumuler des gènes de résistance à des insectes, des champignons, des bactéries ou des virus, ou encore l'adaptation à des stress abiotiques, tels que par exemple la résistance aux températures extrêmes, c'est-à-dire des températures inférieures à 5°C ou supérieures à 30°C, ou encore la résistance à une basse luminosité.

Il est notamment avéré que chez *Lactuca sativa* (laitue de culture), il est impossible ou très difficile de cumuler certains gènes de résistance dans une lignée homozygote, les gènes en question étant situés sur le même locus (un sur chaque allèle), ou sur des locus très proches.

L'obtention de laitues de culture hybrides F1 présenterait un grand intérêt notamment en permettant la production à échelle commerciale, d'hybrides cumulant plusieurs gènes d'intérêt agricole co-dominants ou dominants portés par les différents allèles d'un même locus ou de locus très proches.

Le genre Lactuca comprend plus de 100 espèces, au nombre desquelles *Lactuca sativa* (espèce cultivée), *Lactuca saligna* (espèce sauvage), *Lactuca serriola* (espèce sauvage) et *Lactuca virosa* (espèce sauvage).

*Lactuca sativa* est une espèce diploïde (2n=18), largement autogame, la production de graines se faisant à 98% par auto-pollinisation. Les étamines (organes mâles) sont groupés et forment un tube staminal dans lequel s'ouvrent les sacs polliniques des anthères. Le pistil (organe femelle) est constitué d'un ovaire, d'un style, et d'un stigmate bifide. A l'ouverture de la fleur, le style s'allonge à l'intérieur du tube staminal, ainsi le stigmate se couvre de pollen et la plante est autofécondée. Une plante *Lactuca sativa* donne généralement 0,5 à 6 grammes, voire jusqu'à 10 grammes de graines selon les conditions de culture, chaque gramme comptant généralement 600 à 1000 graines.

Une technique classique pour la production de végétaux hybrides à grande échelle consiste à cultiver les deux variétés utilisées en tant que « parents » à proximité l'une de l'autre et à utiliser des insectes pour la pollinisation, l'un des parents portant une stérilité mâle pour éviter la contamination due à l'auto-pollinisation. Les graines sont ensuite récoltées sur le parent mâle-stérile.

Cette technique permet de cumuler des gènes d'intérêt présents sous forme homozygote dans chacun des parents tout en conservant une homogénéité de parcelle, la génération F1 présentant un phénotype homogène à 100% au stade de la récolte (avant l'ouverture des fleurs).

Il s'avère qu'il est très difficile de produire des laitues de culture *(Lactuca sativa)* hybrides à grande échelle par cette technique. En effet, la fleur de *Lactuca sativa* ne s'ouvre qu'à une seule reprise, pendant une durée de quelques heures seulement, par exemple de une à quatre heures, à un moment de la journée (tôt le matin) durant lequel les insectes pollinisateurs classiques tels que certaines espèces d'abeilles *(Apis mellifera)* ou de bourdons (Bombus spp.) ne sont pas en activité.

La pollinisation de *Lactuca sativa* ne se fait pas non plus par l'intermédiaire du vent.

L'identification d'un insecte pollinisateur pouvant être utilisé pour la production de laitues hybrides se heurte ainsi à de nombreuses difficultés, notamment :
- la durée d'ouverture des fleurs est à la fois courte et matinale, ce qui rend difficile l'identification d'un insecte visitant ces fleurs ;
- les fleurs ne s'ouvrent qu'une seule fois, en conséquence l'insecte pollinisateur choisi doit être un insecte visitant les fleurs tous les jours afin d'être certain qu'un nombre maximum de fleurs mâles stériles soit pollinisé.

Par ailleurs, la production d'hybrides F1 nécessite de disposer d'une lignée « mâle » et d'une lignée « femelle ».

La lignée « femelle » peut être obtenue par le biais de la castration manuelle de la plante. Or, dans le cas de la laitue, chaque fleur ne s'ouvrant qu'à une seule reprise et pour une durée très courte (quelques heures), cette méthode peut difficilement être mise en oeuvre à échelle commerciale du fait de la main d'oeuvre très importante qu'elle nécessiterait.

La lignée « femelle » peut également être obtenue par le biais de l'introduction d'une stérilité de type génique (ou nucléaire) ou cytoplasmique, se manifestant par une absence d'anthères, des anthères vides ou du pollen non viable. Cette stérilité est transmise à la descendance partiellement dans le cas d'une stérilité nucléaire (ou génique), ou totalement dans le cas d'une stérilité cytoplasmique.

Dans leurs travaux, Goubara & Takasaki (Appl. Entomo. Zool. 38(4) : 571-581, 2003 [1]) ont mené des expériences en champ ouvert et fermé visant à identifier des insectes pollinisateurs potentiels pouvant être utilisés pour la production de laitues *Lactuca sativa* hybrides. Parmi 22 insectes observés (21 espèces d'abeilles et une espèce de mouche se nourrissant de nectar, *Syrphidae Eristalis tenax),* l'abeille *Lagioglossum villosulum trichopse* a été retenue comme le meilleur pollinisateur potentiel. Goubara & Takasaki (Appl. Entomo. Zool. 39(1) : 163-169, 2004 [2]) ont également mené des essais d'hybridation à petite échelle entre une laitue portant un gène de stérilité mâle et une laitue mâle-fertile en milieu fermé en présence de l'abeille *Lagioglossum villosulum trichopse.* Les auteurs déclarent avoir obtenu des laitues F1 hybrides, mais avec un très faible rendement.
Aucune autre expérience de production de *Lactuca sativa* hybride utilisant un insecte pollinisateur n'est connue à cette date.

A ce jour, aucune variété de laitue hybride F1 n'est produite à une échelle commerciale.

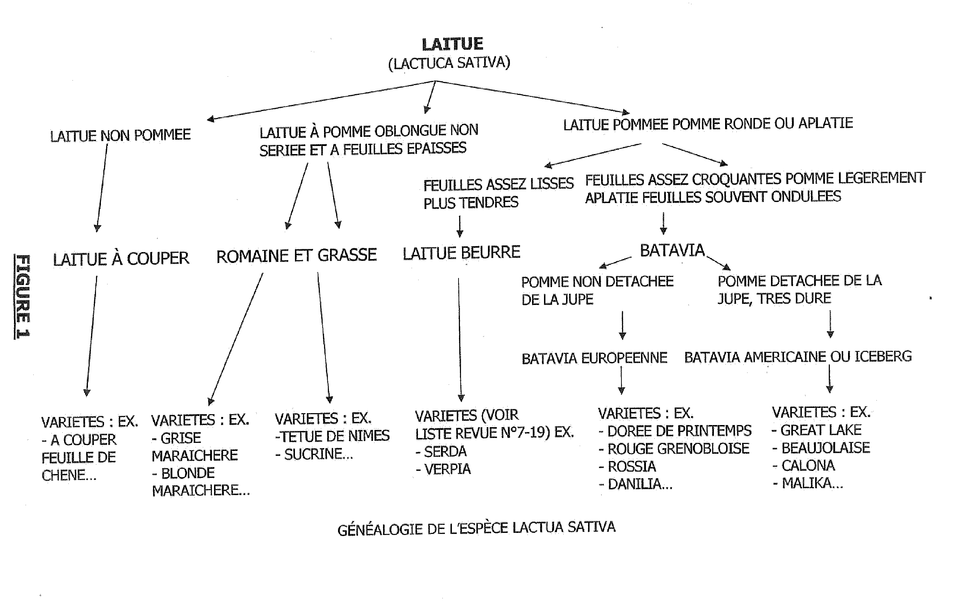

Selon l'invention, les termes suivants s'entendent comme suit :
- Par laitues de culture, on entend l'espèce *Lactuca sativa.* Il existe cinq cultigroupes principaux de laitues cultivées (voir figure 1) _{:} *Lactuca sativa* var. *angustana* (laitue asperge) ; *Lactuca sativa* var. *capitata* (laitue pommée, laitue beurre) ; *Lactuca sativa* var. *crispa* (laitue Batavia ou Iceberg) ; *Lactuca sativa* var. *longifolia* (laitue romaine) et *Lactuca sativa* var. acephala (laitue frisée, laitue à couper). L'invention englobe l'utilisation de chacun de ces différents types de laitues.

- Par pollinisation, on entend le fait de transporter le pollen de l'anthère au stigmate de la même fleur ou d'une autre fleur. Ce système sexué est le mode de reproduction privilégié des plantes à fleurs (angiospermes et gymnospermes). Il permet au grain de pollen d'atteindre le stigmate, puis de former à travers le style un tube pollinique menant jusqu'à l'ovule afin de le féconder.
- Par auto-pollinisation, on entend la pollinisation d'un individu ou d'un biotype par son propre pollen, les individus qui en résultent sont dits auto-pollinisés.
- Le terme autogamie désigne la capacité d'une plante à s'autoféconder, les deux gamètes étant issus du même individu.
- Le terme allogamie désigne le phénomène suivant lequel les fleurs d'un individu sont fécondées par du pollen provenant d'un ou de plusieurs autres individus.
- Par insecte pollinisateur, on entend un insecte (entre autres les abeilles, les papillons, les diptères ou certains coléoptères) qui, explorant les fleurs (par exemple à la recherche de nectar) se frotte aux étamines, récoltant ainsi quelques grains de pollen qu'il abandonnera par la suite sur une autre fleur.

- Le terme locus désigne l'emplacement qu'occupe un gène ou un allèle sur un chromosome.
- Par allèles, on entend les variantes au sein d'une espèce, d'un gène situé à un emplacement chromosomique donné (locus). Différents allèles d'un gène donnent lieu à différentes expressions d'un caractère.
- Par cluster, on entend deux gènes ou plus positionnés à proximité les uns des autres sur le même chromosome. Les clusters de gènes de résistance identifiés chez la laitue ont notamment été décrits par Kesseli et al. [12].
- Par gène dominant, on entend un gène qui confère un phénotype, qu'il soit présent sur les deux chromosomes de la paire ou sur un seul.
- Par gène récessif, on entend un gène qui ne confère un phénotype que lorsqu'il est présent sur chacun des deux chromosomes homologues.
- Par codominance, on entend la propriété de deux gènes allèles d'exprimer l'un et l'autre, dans le phénotype, les caractères qu'ils déterminent. Chez un sujet hétérozygote porteur de deux gènes allèles codominants, le génotype sera complètement exprimé dans le phénotype en ce qui concerne l'information portée par ces gènes.
- Par hétérozygote, on entend une cellule ou un individu qui possède deux gènes allèles distincts sur un locus déterminé de la même paire de chromosomes.
- Par homozygote, on entend une cellule ou un individu qui possède deux gènes allèles identiques sur un locus déterminé de la même paire de chromosomes.

- Par hybride, on entend le produit du croisement entre individus de constitution génétique différente, de préférence d'une même espèce.
- Par hybride F1, on entend la première génération issue d'un croisement entre individus de constitution génétique différente. En conséquence, les hybrides F1 sont hétérozygotes pour au moins un gène.
- Par rétrocroisement (ou croisement en retour, ou « backcross »), on entend le croisement entre un hybride et l'un de ses parents.
- Par cultivar, on entend une variété.

- Par génotype, on entend l'ensemble du matériel génétique porté par un individu, et qui constitue son patrimoine héréditaire.
- Le terme phénotype désigne l'ensemble des caractères morphologiques ou fonctionnels apparents d'un individu, qui correspondent à la fois à la partie exprimée du génotype et à des phénomènes déterminés par le milieu extérieur.
- Par phénotype d'intérêt agricole, on entend un phénotype, issu par exemple d'un croisement entre deux génotypes homozygotes, présentant des caractéristiques intéressantes du point de vue agricole, telles que, entre autres, le cumul de caractères de résistance à différents pathogènes ou insectes, la vigueur hybride (c'est-à-dire le niveau moyen d'un caractère de l'hybride étant supérieur au niveau moyen des deux parents), l'homéostasie, la capacité d'adaptation à des stress abiotiques, les caractéristiques morphologiques telles que la couleur, la forme, le caractère souple ou rigide des feuilles, la composition en nutriments ou les qualités gustatives de la plante.

- Par hétérosis ou vigueur hybride, on entend le phénomène selon lequel un hybride F1 est significativement supérieur au meilleur de ses parents quant à un ou plusieurs caractères, notamment pour ce qui concerne la vigueur.
- Par homéostasie, on entend la capacité d'une plante à s'adapter à son environnement, voire à plusieurs caractéristiques environnementales.

- Le terme mâle stérile désigne une plante incapable de se reproduire par autopollinisation, du fait d'une stérilité des éléments mâles des fleurs. Par exemple, le pollen peut ne pas être fonctionnel, ou il peut y avoir des anomalies structurelles des organes de reproduction mâles, par exemple au niveau du tapis nourricier des anthères (tapetum).
- Par stérilité mâle cytoplasmique, on entend une stérilité qui se transmet de manière homogène par la mère selon une hérédité de type cytoplasmique.
- Par stérilité mâle nucléaire (ou génique), on entend une stérilité à hérédité mendélienne portée par l'ADN du noyau, qui peut être soit sous la dépendance d'un gène récessif, soit sous la dépendance d'un gène dominant.
- Par stérilité mâle monogénique, on entend une stérilité mâle portée par un seul gène.
- Par stérilité mâle plurigénique, on entend une stérilité mâle portée par plusieurs gènes.

- Par résistance, on entend la capacité d'une variété à restreindre la croissance et le développement d'un pathogène ou d'un ravageur déterminé et/ou les dommages qu'ils occasionnent , en comparaison avec des variétés sensibles et dans des conditions similaires, environnementales et de pression de ce pathogène ou de ce ravageur. Ces variétés peuvent, cependant, exprimer quelques symptômes de la maladie ou quelques dommages en cas de forte pression de ce pathogène ou de ce ravageur.
- Par résistance standard ou haute, on entend la capacité d'une variété à restreindre fortement la croissance et le développement d'un pathogène ou d'un ravageur déterminé dans des conditions de pression normales de ceux-ci, en comparaison avec des variétés sensibles. Ces variétés peuvent, cependant, exprimer des symptômes ou des dommages en cas de forte pression de ce pathogène ou de ce ravageur.
- Par résistance intermédiaire ou modérée, on entend la capacité d'une variété à restreindre la croissance et le développement d'un pathogène ou d'un ravageur déterminé mais pouvant exprimer plus de symptômes ou de dommages en comparaison avec des variétés de résistance haute/standard. Les variétés de résistance intermédiaire montreront des symptômes ou des dommages moins sévères que ceux observés sur des variétés sensibles, en conditions similaires, environnementales et/ou de pression du pathogène ou du ravageur.

- Par marqueur moléculaire, on entend un fragment spécifique d'ADN pouvant être identifié au sein du génome complet d'un individu et pouvant être utilisé pour localiser un gène d'intérêt ou vérifier si un individu a hérité d'une caractéristique particulière d'un organisme parent. Il peut s'agir ou non d'une séquence codante. Dans un croisement génétique, le gène d'intérêt restera généralement lié au marqueur moléculaire. La détection du marqueur moléculaire permet alors de sélectionner les individus présentant le gène d'intérêt sans qu'il soit nécessaire de connaître la séquence de ce gène.

Dans le domaine agronomique, l'emploi de marqueurs moléculaires permet de tester rapidement les plantes en cours de sélection et de retenir celles qui possèdent les caractéristiques recherchées. Dans certains cas, la présence de marqueurs associés à un caractère, permet au sélectionneur de s'affranchir de certains tests ou observations phénotypiques. En particulier, l'emploi d'un marqueur moléculaire spécifique d'un gène de stérilité mâle, préférentiellement d'un gène dominant de stérilité mâle, permet une sélection précoce, avant la floraison, des plantes mâles stériles. Cette sélection permet d'éliminer les plantes mâles fertiles pouvant faire partie de la population de plantes utilisées en tant que parents supposément « femelles » (mâles stériles) pour la production de graines hybrides, par exemple dans le cas où un gène dominant de stérilité mâle est utilisé, et par conséquent de réduire le risque de contamination lié à l'autofécondation de ces plantes mâles fertiles.

La présente invention a pour objet des graines, plantes et cellules de plantes *Lactuca sativa* hybrides caractérisées en ce qu'elles présentent un génotype mâle stérile, et sont hétérozygotes pour au moins un gène non impliqué dans la stérilité mâle et conférant un phénotype détectable à la plante.

La stérilité mâle portée par les graines, plantes ou cellules de plantes *Lactuca sativa,* peut être d'origine nucléaire ou cytoplasmique. Dans le cas où elle est cytoplasmique, la stérilité est transmise par le parent femelle. Elle est souvent due à une interaction entre les mitochondries présentes dans le cytoplasme et des gènes nucléaires. La stérilité mâle cytoplasmique se caractérise par l'apparition d'une descendance présentant un phénotype à 100% mâle stérile en l'absence d'un gène restaurateur de fertilité dans le génome du parent mâle. La fertilité mâle peut être restaurée au niveau de la descendance par la présence d'un gène restaurateur de fertilité (Rf) dans le génome du parent « mâle ».

Plus particulièrement, l'invention concerne le cas où le génotype mâle stérile implique au moins un gène nucléaire et où la graine, la cellule ou la plante est hétérozygote pour le ou les gènes à l'origine de la stérilité mâle.

La stérilité mâle nucléaire est causée par un ou plusieurs gènes de stérilité mâle transmis par l'ADN du noyau, ce ou ces gènes pouvant être dominants ou récessifs.

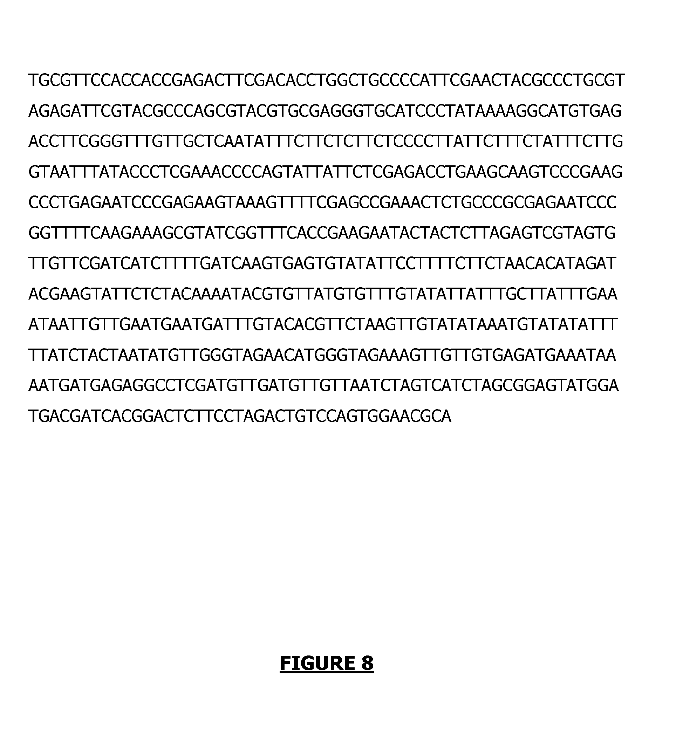

Au nombre de ces gènes de stérilité mâle, on compte notamment le gène dominant nucléaire Ms7 décrit par Ryder (J.Amer.Soc.Hort.Sci 96(6) 826-828, 1971 [8]). Les plantes mâles stériles sont hétérozygotes, Ms7ms7. Les plantes fertiles sont homozygotes ms7ms7. L'obtention des homozygotes dominants Ms7Ms7 reste très difficile voir impossible. De plus, selon Ryder, le caractère dominant de cette stérilité mâle constitue un frein à l'utilité de ce gène pour la production de laitues hybrides. Un échantillon de graines *Lactuca sativa* obtenues selon le procédé de la présente invention, déposé au NCIMB (NCIMB Ltd., Ferguson Building, Craibstone Estate, Bucksburn, Aberdeen, A21 9YA, Scotland, UK) le 13 février 2007 sous le numéro d'accession NCIMB 41470, constitue une source du gène Ms7. Par ailleurs, le marqueur moléculaire RAPD BA05-675 (SEQ ID 2) développé par les inventeurs a un intérêt tout particulier dans l'aide à la sélection des plantes de laitues mâles stériles Ms7, dans la mesure où il permet la mise en place d'un test moléculaire à un stade précoce avant floraison afin d'identifier les plantes mâles stériles qui serviront au croisement suivant. La détection de ce marqueur dont la séquence est présentée en figure 8, permet d'identifier une plante mâle stérile dans 96% des cas en moyenne, toutes typologies de laitue confondues (tableau 18).

Trois gènes de stérilité mâle récessifs, ms1, ms2 et ms3, ont également été identifiés par Lindqvist (Heriditas 46 :387-470, 1960 [3]), et trois autres, ms4, ms5 et ms6, par Ryder (Proc. Am. Soc. Hort. Sci 83 : 585-595, 1963 [6], Proc. Am. Soc. Hort. Sci 91 : 366-368, 1967 [7]). Cependant, dans son ouvrage publié en 1979 (Leafy Salad Vegetables, p. 30 [9]), Ryder déclare que les gènes de stérilité ms1, ms2, ms3, ms4, ms5, ms6 et Ms7 étant tous nucléaires, il est peu probable qu'ils puissent être utilisés pour produire des hybrides F1. En dépit de cela, les inventeurs ont obtenu avec succès des graines, plantes et cellules hybrides en utilisant une stérilité mâle d'origine nucléaire.

La stérilité mâle nucléaire peut être plurigénique ou monogénique. Dans le cas où elle est plurigénique, elle peut par exemple être obtenue au moyen d'un complexe de gènes récessifs, par exemple en cumulant les gènes ms1, ms2 etms3 cités plus haut. L'invention concerne préférentiellement le cas où la stérilité mâle est monogénique.

La stérilité mâle nucléaire peut être dominante ou récessive. En particulier l'invention concerne le cas où le gène de stérilité mâle est monogénique dominant. Plus particulièrement encore, l'invention concerne le cas où la stérilité mâle est conférée par le gène dominant Ms7 cité plus haut.

De préférence, le génome des graines, plantes ou cellules de l'invention comporte une séquence ADN double brin de 650 à 700 nucléotides, par exemple 655 à 695, 660 à 690, 665 à 685, 670 à 680, 673 à 677 nucléotides, ou encore 674, 675 ou 676 nucléotides, dont les extrémités 5' de chacun des deux brins débutent par la séquence « 5' TGCGTTCCAC 3' » (SEQ ID N°1). Selon une mise en oeuvre préférée le génome des graines, plantes ou cellules de l'invention comporte la séquence nucléotidique illustrée à la figure 8 (SEQ ID N°2) ou une séquence dérivée de cette séquence dans laquelle 1 à 10, préférentiellement 1 à 5 ou encore 1 à 3 nucléotides ont été remplacés par d'autres, supprimés ou ajoutés.

L'invention concerne préférentiellement le cas où le phénotype détectable conféré par le ou les gènes hétérozygotes est un phénotype d'intérêt agricole, tel que par exemple, la résistance à différents pathogènes ou insectes, la vigueur hybride (ou hétérosis), l'homéostasie (stabilité de la plante dans différents environnements), l'adaptation à des stress abiotiques tels que par exemple des températures extrêmes ou de basses luminosités, un rendement supérieur à celui des variétés dont est issu un hybride, les caractéristiques morphologiques telles que la couleur, la forme, la taille, le caractère souple ou rigide des feuilles, la composition en nutriments ou les qualités gustatives de la plante.

De plus, les inventeurs ont observé que de façon surprenante, les plantes hybrides issues de graines selon l'invention issues de divers croisements entre différentes laitues, cultivées en période hivernale et en région tempérée avaient une croissance plus rapide, arrivant à maturité en moyenne 7 à 10 jours plus tôt que les plantes parentes cultivées dans les mêmes conditions. Il a également été observé que l'homogénéité de développement intraparcelle des hybrides F1 (sur des effectifs de 30 plantes environ par parcelle) est supérieure à celle des parcelles de lignées (plantes parentes). En d'autres termes, le nombre de plantes présentant un retard ou une avance dans leur développement par rapport à l'ensemble des plantes d'une même parcelle est nettement plus faible sur les parcelles d'hybrides F1 que sur les parcelles de variétés lignées. Par conséquent ces caractéristiques des hybrides F1 permettent non seulement de raccourcir les cycles de production de laitue mais aussi de condenser la récolte sur une durée plus courte.

Le phénotype d'intérêt agricole peut notamment être conféré par un ou plusieurs gènes de résistance standard ou intermédiaire à une infection par un virus, une bactérie, un insecte ou un champignon, et plus particulièrement encore à l'un des champignons suivants : *Brémia lactucae, Fusarium oxysporum, Sclérotinia* minor ou *sclérotorum, Botrytis cinerea, Rhizictonia solani, Microdochium panattonianum, Verticiulium dahliae, Erysiphe chicocearum* ou *Pithium tracheiphilum;* à l'un des insectes suivants : *Nasonovia ribisnigri, Myzus persicae, Macrosiphum euphorbia, Nématodes pratylenchus ou meloidogyne,* mineuses *Liriomyza huidobrensis ou Pemphigus busarius;* à l'une des bactéries suivantes : pseudomonas, xanthomonas ou rhizomonas ; ou encore à l'un des virus suivants : LMV (virus de la mosaïque de la laitue), TSWV (virus des tâches bronzées de la tomate), « Big vein » (ou maladie des grosses nervures composé des virus LBVV (virus de la grosse nervure de la laitue) et MILV(Virus Mirafiori de la laitue)), TBSV (virus du rabougrissement buissonneux de la tomate), LNSV (virus de la tâche nécrotique de la laitue), TuMV (virus de la mosaïque du navet), CMV (virus de la mosaïque du concombre) ou BWYV (virus de la jaunisse de la betterave).

Dans le cas ou le phénotype d'intérêt agricole est conféré par un ou plusieurs gènes de résistance standard ou intermédiaire à une infection par un virus, une bactérie, un insecte ou un champignon, le ou lesdits gènes de résistance standard ou intermédiaire conférant ce phénotype peuvent notamment être choisis parmi les gènes de résistance au brémia Dm10, R17, Dm5, Dm8, R36, R37 (gènes situés sur le cluster 1 de *Lactuca sativa*),Dm1, Dm2, Dm3, Dm6, Dm14, Dm15 Dm16, Dm18 (gènes situés sur le cluster 2 de *Lactuca sativa),* Dm4, Dm7, Dm11, R38 (gènes situés sur le cluster 4 de *Lactuca sativa) ;* ou encore le gène Tu de résistance au TuMV situé sur le cluster 1; le gène Nr de résistance au Nasonovia situé sur le cluster 2; ou parmi les gènes mol.1 et mol.2 de résistance au LMV situés sur le cluster 4. Les clusters 1, 2 et 4 cités ci-dessus sont notamment définis par Michelmore R.W. (Plant Pathol., 1987, vol. 36, no4 : 499-514 **[4]**, Theor. Appl. Genet., 1993, vol. 85, n°8 : 985-993 **[5])**.

Le phénotype d'intérêt agricole peut plus particulièrement être conféré par un ou plusieurs gènes de résistance standard ou intermédiaire à l'une des principales maladies touchant la laitue, le *Bremia lactucae,* un champignon qui provoque sur la face intérieure du limbe un duvet blanchâtre et poudreux. Le Bremia a de plus un fort potentiel d'adaptation qui conduit à l'apparition de nouvelles races capables de contourner les résistances déjà introduites par les sélectionneurs dans les variétés.

L'invention concerne préférentiellement encore le cas où les graines, plantes ou cellules de plantes *Lactuca sativa* sont hétérozygotes pour au moins deux gènes non impliqués dans la stérilité mâle et conférant un phénotype détectable à la plante. Encore plus préférentiellement, les deux gènes ou plus conférant un phénotype détectable à la plante sont situés sur un même cluster, par exemple sur un cluster de gènes de résistance tel que le cluster 1, 2 ou 4 de *Lactuca sativa.*

L'invention a également pour objet une population de graines hybrides *Lactuca sativa* présentant un génotype mâle stérile, et hétérozygote pour au moins un gène non impliqué dans la stérilité mâle et conférant un phénotype détectable aux plantes issues de ces graines, présentant éventuellement les caractéristiques additionnelles des graines décrites ci-dessus, telle que ladite population comprend au moins 10⁵ graines, de préférence au moins 10⁶, et encore plus préférentiellement au moins 10⁷ graines.

Dans le cas où les graines sont obtenues par croisement entre deux plantes *Lactuca sativa,* dont l'une (parent « femelle ») est porteuse d'une stérilité mâle nucléaire conférée par un ou plusieurs gènes dominants, et que les plantes utilisées en tant que parents « femelles » sont elles-mêmes issues d'un croisement, les plantes utilisées en tant que parents « femelles » sont forcément hétérozygotes pour le gène de stérilité. En effet, pour qu'elles soient homozygotes pour le ou les gènes de stérilité mâles dominants, il faudrait que les deux plantes dont sont issues par croisement les plantes utilisées en tant que parents « femelles » soient elles-mêmes porteuses du gène de stérilité mâle, or si cela était le cas un croisement entre ces deux plantes serait impossible.

En conséquence, la population de graines selon l'invention issues du croisement entre deux types de plantes *Lactuca sativa,* dont l'un (parent « femelle ») est porteur d'une stérilité mâle nucléaire conférée par un ou plusieurs gènes dominants, est composée, en raison du phénomène de ségrégation des chromosomes lors de la méiose, de graines *Lactuca sativa* porteuses du ou des gènes dominants de stérilité mâle, et de graines non porteuses de ce ou ces gènes. Dans le cas où la stérilité mâle portée par le parent « femelle » est monogénique et dominante, la proportion de graines mâle stériles est généralement d'au moins 40%.

La présente invention a notamment été réalisée suite à l'observation par les inventeurs du fait que, bien que les diptères ne soient pas des insectes pollinisateurs habituels des fleurs de *Lactuca sativa,* et ne soient pas non plus connus comme se nourrissant de leur nectar, ces insectes, notamment les espèces *Calliphora vomitaria* ,*Calliphora erythrocephala,* et *Lucilia caesar,* quand introduits en surnombre dans un milieu fermé, agissent comme pollinisateurs de *Lactuca sativa.*

Aussi, la présente invention a également pour objet l'utilisation d'insectes de l'ordre des diptères pour effectuer la pollinisation dans un milieu fermé, de plantes *Lactuca sativa* mâle stériles, par des plantes mâles fertiles, notamment en vue d'obtenir des plantes *Lactuca sativa* hybrides. Préférentiellement, les plantes *Lactuca sativa* mâles stériles utilisées possèdent une stérilité mâle portée par un unique gène dominant, de préférence la stérilité mâle Ms7 cité plus haut. Les plantes mâles fertiles utilisées sont préférentiellement des plantes *Lactuca sativa,* et encore plus préférentiellement préférence un cultivar.

Le milieu fermé selon l'invention peut notamment être une serre, une cage ou un tunnel, d'une superficie supérieure de préférence à 30 m², encore plus préférentiellement à 300 m², par exemple de 30 à 1500 m² ou 50 à 1000 m². La hauteur du milieu fermé est normalement comprise entre 2m et 4m, préférentiellement entre 2,5m et 3,5m par exemple 3m. Il peut notamment comporter des moyens de ventilation, d'arrosage, de contrôle de la température et de la luminosité. De préférence, ledit milieu fermé est une enceinte hermétique aux insectes.

Les diptères utilisés doivent préférentiellement être présents à une concentration d'au moins 100 diptères par m², de préférence au moins 250 diptères par m², par exemple 100 à 1000 diptères par m². La concentration peut également être déterminée en nombre de diptères par m³, et est préférentiellement d'au moins 25 diptères par m³, préférentiellement d'au moins 50 diptères par m³, préférentiellement encore d'au moins 75 diptères par m³, par exemple de 25 à 500 diptères par m³ ou de 75 à 250 diptères par m³.

La présente invention a également pour objet un procédé d'obtention de graines *Lactuca sativa* hybrides, comprenant :
- une étape de culture dans un milieu fermé, de plantes *Lactuca sativa* phénotypiquement mâles stériles utilisées en tant que parents « femelles » et de plantes Lactuca *sativa* phénotypiquement mâles fertiles utilisées en tant que parents « mâles », à proximité les unes des autres, l'un des deux parents présentant comme caractéristique supplémentaire le fait d'être homozygote pour un gène lui conférant un phénotype détectable autre que la stérilité mâle, l'autre parent ne portant pas ce gène ;
- une étape de pollinisation par des diptères introduits dans le milieu fermé à l'époque de la floraison des plantes à une concentration supérieure à 100 diptères par m², de préférence au moins 250 diptères par m² ; et
- une étape de récolte des graines produites par les plantes mâles stériles.

Préférentiellement, le second parent est également homozygote pour au moins un gène lui conférant un phénotype détectable autre que la stérilité mâle, non porté par l'autre parent. De préférence, ledit milieu fermé est une enceinte hermétique aux insectes.

Il a été constaté de meilleurs résultats lorsque les diptères sont en surnombre, aussi, leur concentration est préférentiellement, supérieure à 400 diptères par m², et encore plus préférentiellement supérieure à 500 diptères par m².

Les diptères peuvent être introduits au stade d'oeufs, de larves, de pupes ou d'adultes. Ils sont de préférence introduits sous forme de pupes.

Les diptères utilisés sont de préférence des brachycères, par exemple des brachycères cyclorhaphes, de préférence encore des brachycères de la famille des Muscidae ou des Calliphorides, par exemple des *Calliphora vomitaria,* des *Calliphora erythrocephala* ou des *Lucilia caesar.*

Un meilleur rendement est normalement obtenu si l'introduction de diptères est renouvelée au moins une fois par semaine, de préférence deux fois par semaine, durant au moins 3 à 4 semaines.

De même, un meilleur rendement est normalement obtenu si le nombre de plantes mâles stériles dans le milieu fermé, est plus grand que le nombre de plantes mâles fertiles. Le nombre de plantes mâles stériles dans le milieu fermé, est par exemple d'au moins 2000, et le nombre de plantes mâles fertiles par exemple d'au moins 1000.

La mise en oeuvre du procédé selon l'invention nécessite qu'il y ait concordance de floraison entre les deux parents. Cette dernière peut être obtenue par sélection sur ce caractère ou par des pratiques culturales appropriées.

De préférence, la stérilité mâle des plantes utilisées en tant que parents femelles est monogénique, dominante et nucléaire, et encore plus préférentiellement conférée par le gène Ms7.

De préférence, le génome des plantes utilisées en tant que parents femelles comporte une séquence ADN double brin de 650 à 700 nucléotides, par exemple 655 à 695, 660 à 690, 665 à 685, 670 à 680, 673 à 677 nucléotides, ou encore 674, 675 ou 676 nucléotides, dont les extrémités 5' de chacun des deux brins débutent par la séquence « 5' TGCGTTCCAC 3' » (SEQ ID N°1). Selon une mise en oeuvre préférée le génome des plantes utilisées en tant que parents femelles comporte la séquence nucléotidique illustrée à la figure 8 (SEQ ID N°2) ou une séquence dérivée de cette séquence dans laquelle 1 à 10, préférentiellement 1 à 5 ou encore 1 à 3 nucléotides ont été remplacés par d'autres, supprimés ou ajoutés.

Comme expliqué plus haut, il n'est pas possible, en raison du phénomène de ségrégation des chromosomes lors de la méiose, d'obtenir par les techniques de croisement classiques, une population homogène de plantes utilisées en tant que parents « femelles » porteuses d'un gène dominant de stérilité mâle. En conséquence, dans le cas où un gène dominant de stérilité mâle est utilisé, les plantes utilisées en tant que parents femelles peuvent être obtenues par un procédé comprenant :
- une étape de croisement entre des plantes *Lactuca sativa* hétérozygotes pour un gène dominant de stérilité mâle nucléaire et des plantes *Lactuca sativa* mâles fertiles ne portant pas de gènes de stérilité,
- une étape de culture des graines issues dudit croisement, et
- une étape d'élimination des plantes présentant un phénotype mâle fertile.

L'étape d'élimination des plantes présentant un phénotype mâle fertile peut par exemple être réalisée manuellement en se basant sur un caractère visible permettant de distinguer les plantes *Lactuca sativa* mâles stériles des plantes *Lactuca sativa* mâles fertiles. Par exemple, dans le cas d'une stérilité liée au gène Ms7, le tri peut être effectué en utilisant le fait que les capitules des plantes mâles stériles restent ouverts plus longtemps que ceux des plantes mâles fertiles, et que les plantes mâles stériles ne présentent pas de pollen.

Selon une mise en oeuvre alternative, l'étape d'élimination des plantes présentant un phénotype mâle fertile est réalisée au moyen de la détection dans un échantillon de chacune des plantes de l'absence d'un marqueur moléculaire spécifique d'un gène dominant de stérilité mâle. Il peut par exemple s'agir d'un marqueur RAPD (Random Amplification of Polymorphic DNA) d'une longueur de 675 paires de bases environ (par exemple 650 à 700, 655 à 695, 660 à 690, 665 à 685, 670 à 680, 673 à 677, 674, 675 ou 676 paires de bases) dont les extrémités 5' de chacun des deux brins débutent par la séquence « 5' TGCGTTCCAC 3' » (SEQ ID N°1), tel que le marqueur BA05-675 (SEQ ID 2) développé par les inventeurs ou d'un marqueur SCAR (Sequenced Characterized Amplified Region Marker), d'un marqueur CAPS (Cleaved Amplified Polymorphic Sequence) ou de tout autre marqueur généralement désignés sous l'appellation STS (Séquence Tagged Site), développé à partir de ce marqueur RAPD. Selon une mise en oeuvre préférée, la détection de la présence ou non d'un marqueur moléculaire spécifique d'un gène dominant de stérilité mâle est réalisée avant la floraison des plantes utilisées en tant que parents femelles, de préférence à un stade précoce de croissance des plantes, par exemple au stade 1 à 5 feuilles, de préférence encore, au stade 1 à 2 feuilles.

De préférence, le marqueur moléculaire utilisé permet de détecter les plantes mâles stériles avec une sensibilité d'au moins 70%, 75%, 80%, 85%, 95%, 98 ou 99%, ou encore de 100%, la sensibilité étant définie comme le rapport entre le nombre de plantes mâles stériles présentant le marqueur moléculaire (vrais positifs) et la somme du nombre de vrais positifs et du nombre de plantes mâles stériles ne présentant pas le marqueur moléculaire (faux négatifs) (voir tableau 17).

De préférence également, le marqueur moléculaire utilisé permet de détecter les plantes mâles stériles avec une spécificité d'au moins 70%, 75%, 80%, 85%, 95%, 98 ou 99%, ou encore de 100%, la spécificité étant définie comme le rapport entre le nombre de plantes mâles fertiles ne présentant pas le marqueur moléculaire (vrais négatifs) et la somme du nombre de vrais négatifs et du nombre de plantes mâles fertiles présentant le marqueur moléculaire (faux positifs) (voir tableau 17).

La MMS (masse de mille semences) des graines obtenues selon le procédé de l'invention est généralement supérieure d'au moins 10%, voire d'au moins 20%, voire même d'au moins 30% à la MMS des graines obtenues par auto-fécondation des plantes *Lactuca sativa* mâles fertiles utilisées en tant que parents « mâles ».

L'invention concerne également une population de graines susceptibles d'être obtenues selon le procédé décrit ci-dessus, et comprenant au moins 10⁵ graines, préférentiellement 10⁶ graines et encore plus préférentiellement 10⁷ graines.

### Description brève des dessins

Figure 1 : Généalogie de *Lactuca sativa*
Figure 2 : disposition des laitues utilisées en tant que parents « mâles » et « femelles » au cours de l'essai 1
Figure 3 : disposition des laitues utilisées en tant que parents « mâles » et « femelles » au cours de l'essai 2
Figure 4 : génotypage des 50 plantes hybrides de l'essai 10 : profil d'amplification du marqueur moléculaire SCW09 après digestion par TaqI. Le marqueur SCW09 marque les gènes Dm6 et Dm18 de résistance au *Bremia lactucae.* A = témoin Dm18+/Dm18+, B = témoin Dm18-/Dm18- ; C = témoin Dm6+ ; D = non digéré par TaqI.
Figure 5 _{:} génotypage des 50 plantes hybrides de l'essai 10 : profil d'amplification du marqueur moléculaire B1 qui marque le gène Dm3 de résistance au *Bremia lactucae.* A = témoin Dm3+ ; B = témoin Dm3-.
Figure 6 _{:} Profil électrophorétique de l'amorce OPBA05 avec le marqueur BA05-675 (flèche). Electrophorèse sur gel d'agarose à 1,0% pendant 1h45 à 190 V. M : marqueur de poids moléculaire 100-bp ladder (Pharmacia Biotech, réf. 27.4001.01)
Figure 7 _{:} Cartographie du marqueur moléculaire BA05-675 associé au locus Ms7 chez la laitue, *Lactuca sativa* (distance calculée suivant la fonction de Kosambi).
Figure 8 _{:} Séquence nucléotidique 5'-3' du marqueur BA05-675 associé à la stérilité mâle Ms7 chez la laitue, *Lactuca sativa.* (SEQ ID 2)

La présente invention porte plus particulièrement sur les aspects suivants :
1. Graine de *Lactuca sativa* hybride, caractérisée en ce qu'elle
   - présente un génotype mâle stérile, et
   - est hétérozygote pour au moins un gène distinct conférant un phénotype détectable à la plante issue de cette graine.
2. Graine selon l'aspect 1, telle que ledit génotype mâle stérile implique au moins un gène nucléaire et que ladite graine est hétérozygote pour le ou les gènes à l'origine de la stérilité mâle.
3. Graine selon l'aspect 2, telle que la stérilité mâle est monogénique.
4. Graine selon l'aspect 3, telle que le gène de stérilité mâle est dominant.
5. Graine selon l'aspect 1, telle que ledit génotype mâle stérile est conféré par le gène de stérilité Ms7.
6. Graine selon l'une quelconque des aspects 1 à 5 caractérisée en ce que son génome comporte une séquence ADN double brin de 650 à 700 nucléotides dont les extrémités 5' de chacun des deux brins débutent par la séquence TGCGTTCCAC (SEQ ID N°1).
7. Graine selon l'une quelconque des aspects 1 à 6, telle que ledit phénotype est un phénotype d'intérêt agricole.
8. Graine selon l'une quelconque des aspects 1 à 7, telle que ledit phénotype est conféré par un gène de résistance standard ou de résistance intermédiaire à une infection par un virus, une bactérie, un insecte ou un champignon.
9. Graine selon l'une quelconque des aspects 1 à 8, telle que ledit phénotype est une résistance standard ou intermédiaire à l'un des champignons suivants : *Brémia lactucae, Fusarium oxysporum, Sclérotinia minor ou sclérotorum, Botrytis cinerea, Rhizictonia solani, Microdochium panattonianum, Verticiulium dahliae, Erysiphe chicocearum* ou *Pithium tracheiphilum,* à l'un des insectes suivants : *Nasonovia ribisnigri, Myzus persicae, Macrosiphum euphorbia, Nématodes pratylenchus* ou *meloidogyne*, mineuses *liriomyza huidobrensis ou Pemphigus busarius,* à l'une des bactéries suivantes : pseudomonas, xanthomonas ou rhizomonas, ou encore à l'un des virus suivants : LMV, TSWV, « Big vein », TBSV, LNSV, TuMV, CMV ou BWYV.
10. Graine selon l'aspect 8 ou 9, telle que ladite graine présente un second gène de résistance standard ou intermédiaire distinct, sous forme hétérozygote.
11. Graine selon l'aspect 8 ou 10, où le ou lesdits gènes de résistance standard ou intermédiaire sont choisis parmi les gènes suivants de résistance au brémia : Dm10, R17, Dm5/Dm8, R36/R37 (cluster 1), Dm1 Dm2, Dm3, Dm6, Dm14, Dm15, Dm16, Dm18 (cluster 2), ou Dm4, Dm7, Dm11, R38 (cluster 4) ; ou encore le gène Tu de résistance au TuMV (cluster 1) ; le gène Nr de résistance au Nasonovia (cluster 2) ; ou les gènes mol.1, mol.2 de résistance au LMV (cluster 4).
12. Plante de *lactuca sativa* hybride, caractérisée en ce qu'elle :
   - présente un génotype mâle stérile hétérozygote, et
   - est hétérozygote pour au moins un gène distinct lui conférant un phénotype détectable.
13. Plante selon l'aspect 12, telle que ledit génotype mâle stérile est d'origine nucléaire et que ladite plante est hétérozygote pour le ou les gènes à l'origine de la stérilité mâle.
14. Plante selon l'aspect 13, telle que la stérilité mâle est monogénique.
15. Plante selon l'aspect 14, telle que le gène de stérilité mâle est dominant
16. Plante selon l'aspect 12, telle que ledit génotype mâle stérile est conféré par le gène de stérilité Ms7.
17. Cellule d'une plante de *lactuca sativa* hybride, caractérisée en ce qu'elle :
   - présente un génotype mâle stérile hétérozygote, et
   - est hétérozygote pour au moins un gène distinct conférant un phénotype détectable à la plante.
18. Cellule selon l'aspect 17, telle que ledit génotype mâle stérile est d'origine nucléaire et que ladite cellule est hétérozygote pour le ou les gènes à l'origine de la stérilité mâle.
19. Cellule selon l'aspect 18, telle que la stérilité mâle est monogénique.
20. Cellule selon l'aspect 19, telle que le gène de stérilité mâle est dominant
21. Cellule selon l'aspect 17, telle que ledit génotype mâle stérile est conféré par le gène de stérilité Ms7.
22. Population de graines selon l'une quelconque des aspects 1 à 11, telle que ladite population comprend au moins 10⁵ graines, de préférence au moins 10⁶.
23. Population de graines selon l'aspect 22, comprenant au moins 10⁷ graines.
24. Population de graines de *lactuca sativa* comprenant au moins 40% de graines selon l'une quelconque des aspects 1 à 11.
25. Population selon l'aspect 24, où les autres graines ne comprennent pas le gène de stérilité mâle.
26. Utilisation d'insectes de l'ordre des diptères à une concentration supérieure à 100 diptères par m² ou à 25 diptères par m³ pour effectuer la pollinisation, dans un milieu fermé, de plantes *Lactuca sativa* mâles stériles, par des plantes mâles fertiles.
27. Utilisation selon l'aspect 26, où les diptères sont présents à une concentration d'au moins 250 diptères par m² ou d'au moins 75 diptères par m³.
28. Utilisation selon l'aspect 26, afin d'obtenir des plantes *Lactuca sativa* hybrides.
29. Utilisation selon l'une quelconque des aspects 26 à 28, où les plantes *Lactuca sativa* mâles stériles possèdent une stérilité mâle portée par un unique gène dominant, de préférence la stérilité mâle Ms7.
30. Utilisation selon l'une quelconque des aspects 26 à 29, où les plantes mâles fertiles sont également des plantes *Lactuca sativa,* de préférence un cultivar.
31. Procédé d'obtention de graines *Lactuca sativa* hybrides, comprenant :
   - une étape de culture dans un milieu fermé, de plantes *Lactuca sativa* phénotypiquement mâles stériles utilisées en tant que parents « femelles » et de plantes *Lactuca sativa* phénotypiquement mâles fertiles utilisées en tant que parents « mâles », à proximité les unes des autres, l'un des deux parents présentant comme caractéristique supplémentaire le fait d'être homozygote pour un gène lui conférant un phénotype détectable autre que la stérilité mâle, l'autre parent ne portant pas ce gène ;
   - une étape de pollinisation par des diptères introduits dans le milieu fermé à l'époque de la floraison des plantes à une concentration supérieure à 100 diptères par m² ou à 25 diptères par m³, de préférence au moins 250 diptères par m² ou au moins 75 diptères par m³; et
   - une étape de récolte des graines produites par les plantes mâles stériles.
32. Procédé selon l'aspect 31, où le second parent est également homozygote pour au moins un gène lui conférant un phénotype détectable autre que la stérilité mâle, non porté par l'autre parent.
33. Procédé selon l'aspect 31 ou 32, où ladite concentration de diptères introduite est supérieure à 400 diptères par m², de préférence supérieure à 500 diptères par m².
34. Procédé selon l'une quelconque des aspects 31 à 33 où lesdits diptères sont introduits sont forme de pupes.
35. Procédé selon l'une quelconque des aspects 31 à 34, où ledit milieu fermé est une enceinte hermétique aux insectes.
36. Procédé selon l'une quelconque des aspects 31 à 35, où lesdits diptères sont des brachycères, de préférence des brachycères cyclorhaphes.
37. Procédé selon l'aspect 36, où lesdits diptères appartiennent à la famille des Calliphorides ou des Muscidae.
38. Procédé selon l'aspect 37, où lesdits diptères sont des *Calliphora* vomitaria, des *Calliphora erythrocephala* ou des *Lucilia caesar.*
39. Procédé selon l'une quelconque des aspects 31 à 38, où dans le milieu fermé, le nombre de plantes mâles stériles est plus grand que le nombre de plantes mâles fertiles.
40. Procédé selon l'une quelconque des aspects 31 à 39, où dans le milieu fermé, le nombre de plantes mâles stériles est d'au moins 2000, et le nombre de plantes mâles fertiles est d'au moins 1000.
41. Procédé selon l'une quelconque des aspects 31 à 40, où l'introduction de diptères est renouvelée au moins une fois par semaine, durant 3 à 4 semaines.
42. Procédé selon l'une quelconque des aspects 31 à 41, où la stérilité mâle des plantes utilisées en tant que parents femelles est d'origine monogénique, dominante et nucléaire, et où les plantes utilisées en tant que parents femelles sont obtenues par un procédé comprenant :
   - une étape de croisement entre des plantes *Lactuca sativa* hétérozygotes pour un gène dominant de stérilité mâle nucléaire et des plantes *Lactuca sativa* mâles fertiles ne portant pas de gènes de stérilité,
   - une étape de culture des graines issues dudit croisement, et
   - une étape d'élimination des plantes présentant un phénotype mâle fertile.
43. Procédé selon l'aspect 42, où l'étape d'élimination des plantes présentant un phénotype mâle fertile est réalisée au moyen de la détection dans un échantillon de chacune des plantes de l'absence d'un marqueur moléculaire spécifique du gène dominant de stérilité mâle.
44. Procédé selon l'aspect 42 ou 43, où la stérilité mâle des plantes utilisées en tant que parents femelles est conférée par le gène Ms7.
45. Procédé selon l'une quelconque des aspects 31 à 44 où le génome des plantes utilisées en tant que parents femelles comporte une séquence ADN double brin de 650 à 700 nucléotides dont les extrémités 5' de chacun des deux brins débutent par la séquence TGCGTTCCAC (SEQ ID N°1)
46. Population de graines susceptibles d'être obtenues selon l'une quelconque des aspects 31 à 45, comprenant au moins 10⁵ graines, préférentiellement 10⁶ graines et encore plus préférentiellement 10⁷ graines.
47. Population de graines selon l'aspect 46, telle que la MMS (masse de mille semences) soit supérieure d'au moins 10%, préférentiellement d'au moins 20% et encore plus préférentiellement d'au moins 30% à la MMS des graines obtenues par auto-fécondation des plantes *Lactuca sativa* mâles fertiles utilisées en tant que parents « mâles ».

### Exemples

### EXEMPLE 1 : OBTENTION DE GRAINES DE LAITUES HYBRIDES

Les plantes *Lactuca sativa* mâles stériles utilisées dans les différents essais décrits ci-dessous en tant que parents « femelles » sont porteuses du gène de stérilité Ms7. Ces plantes sont issues d'un rétrocroisement de 5^{ème} génération (BC5) d'une laitue beurre d'abri porteuse du gène Ms7 (X Girelle 94-9538-1) pouvant être obtenue auprès de divers organismes de recherche tels que l'INRA, l'USDA, etc.

Le gène Ms7 étant dominant, un croisement entre deux plantes porteuses du gène Ms7 est impossible. En conséquence, les plantes présentant un phénotype de stérilité mâle sont forcément hétérozygotes pour le gène Ms7 et ne peuvent être obtenues que par le biais du croisement entre des plantes Ms7/ms7 (stériles) et ms7/ms7 (fertiles).

Il en résulte qu'une étape d'épuration est nécessaire pour éliminer les plantes mâles fertiles. Cette dernière est rendue possible par le fait que les plantes Ms7/ms7 n'ont pas de pollen et que les capitules des plantes Ms7/ms7 restent ouverts plus longtemps que ceux des plantes ms7/ms7.

### Essai 1

Dans le cadre de ce premier essai :
- 450 plantes *Lactuca sativa* beurres d'abri issues d'un rétrocroisement de 3^{ème} génération (BC3) des laitues beurres Ms7 avec des laitues beurres du cultivar Nacre/Cambria Dm18/R38 (gènes de résistance Dm18 du cluster 2 et R38 du cluster 4), une combinaison sensible au *Brémia lactucae* race 24 (Bl24), ont été utilisées en tant que parents « femelles » (environ la moitié d'entre elles étant mâles stériles); et
- 100 plantes *Lactuca sativa* du cultivar beurre d'abri BRA Dm18/R37 (gènes de résistance Dm18 du cluster 2 et R37 du cluster 1), une combinaison résistante à B124, ont été utilisées en tant que parents « mâles ».

Les deux géniteurs présentent une concordance parfaite de floraison ce qui permet une fécondation la plus homogène possible.

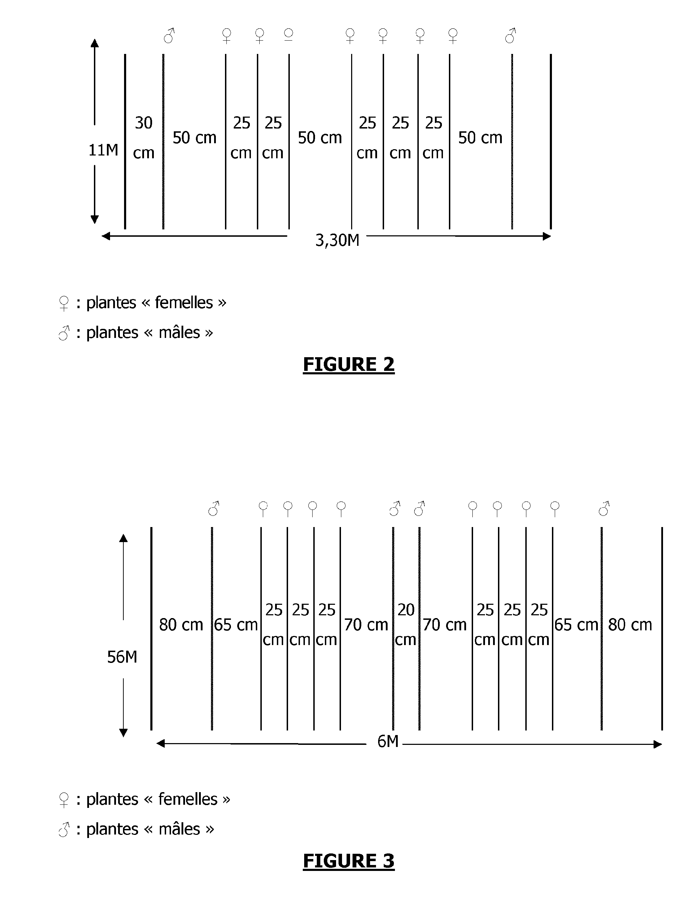

Les plantes ont été disposées dans une enceinte fermée hermétique aux insectes de 36 m² (serre de type hollandaise avec possibilité de contrôle de l'hygrométrie et de la température, 11m x 3,30 m) de la manière suivante (voir figure 2) :
- les plantes utilisées en tant que parents « mâles », ont été disposées en deux rangs le long des bords de l'enceinte fermée, les plantes étant espacées de 20 cm, et
- les plantes utilisées en tant que parents « femelles » ont été disposées en sept rangs entre les deux rangs de plantes « mâles », à une distance de 50cm de ces dernières, les plantes étant espacées de 15cm, et les rangs de 25 cm.

Les semis ont été effectués en semaine 13 et les plantations en semaine 16.

Du fait du caractère dominant du gène Ms7, une étape d'épuration (élimination) des plantes utilisées comme parents femelles présentant un phénotype mâle fertile a été nécessaire. Cette étape a été réalisée manuellement en semaines 26 et 27 en utilisant le fait que les plantes Ms7/ms7 n'ont pas de pollen et que les capitules des plantes Ms7/ms7 (mâles stériles) restent ouverts plus longtemps que ceux des plantes ms7/ms7 (mâles fertiles).

Sur les 450 plantes utilisées en tant que parents femelles, 217 se sont révélées mâles stériles et ont été effectivement utilisées.

Deux types d'insectes de l'ordre des Diptères et de la famille des Calliphorides ont été utilisés comme insectes pollinisateurs :
- des mouches *Calliphora vomitaria* et *Calliphora erythrocephala* dites 'asticots', (aussi appelées mouches bleues ou mouches de la viande) travaillant à 15-20 °C, et
- des mouches *Lucilia caesar* dites 'pinkies' (aussi appelées mouches vertes des cadavres), travaillant à 20-25°C.

Les apports en insectes pollinisateurs ont été réalisés à fréquence bi-hebdomadaire durant les semaines 28 à 31 à raison de 300 mouches par m² par apport.

La récolte des plantes mâles stériles (parents « femelles ») et des plantes mâles fertiles (parents « mâles ») utilisées aussi comme témoin d'autofécondation a été réalisée en semaine 33.

Les plantes ont été séchées, battues, puis les graines ont été passées sur une colonne à air pulsé pour parfaire le nettoyage et éliminer les déchets légers résiduels et calibrées en longueur et en largeur.

Un échantillon de 2500 graines obtenues lors de l'essai 1 a été déposé le 13 février 2007 au NCIMB (NCIMB Ltd., Ferguson Building, Craibstone Estate, Bucksburn, Aberdeen, A21 9YA, Scotland, UK) sous le numéro d'accession NCIMB 41470.

### Essai 2

Un second essai similaire a été réalisé à plus grande échelle dans une enceinte fermée, hermétique aux insectes, de 336 m² (longueur : 56 m, largeur : 6 m, hauteur : 3 m).

Dans le cadre de ce deuxième essai :
- 3000 plantes *Lactuca sativa* beurres d'abri issues d'un rétrocroisement de 4^{ème} génération (BC4) des laitues beurres Ms7 avec des laitues beurres du cultivar Nacre/Cambria Dm18/R38 en Brémia (combinaison sensible au B124), ont été utilisées en tant que parents « femelles » (environ la moitié d'entre elles étant mâles stériles); et
- 1500 plantes *Lactuca sativa* d'un cultivar beurre d'abri BRA Dm18/R37 résistantes à B124 ont été utilisées en tant que parents « mâles ».

Les plantes ont été disposées dans l'enceinte fermée de la manière suivante (voir figure 3) :
- les plantes utilisées en tant que parents « mâles » ont été disposées en quatre rangs : un rang le long de chaque bord de l'enceinte fermée (à 80 cm) et deux rangs au centre (espacés de 20 cm), les plantes étant disposées en quinconce et espacées de 15 cm, et
- les plantes utilisées en tant que parents « femelles » ont été disposées en huit rangs entre les rangs de plantes mâles fertiles, à 65 et 70 cm de ces derniers, les plantes étant espacées de 15cm, et les rangs de 25 cm.

Les semis ont été effectués en semaine 12 et les plantations en semaine 16.

Similairement à ce qui a été effectué lors le l'essai 1, une étape d'épuration (élimination) des plantes utilisées comme parents femelles présentant un phénotype mâle fertile a été nécessaire. Cette étape a été réalisée manuellement en semaines 25 et 26 en utilisant le fait que les plantes Ms7/ms7 n'ont pas de pollen et que les capitules des plantes Ms7/ms7 (mâles stériles) restent ouverts plus longtemps que ceux des plantes ms7/ms7 (mâles fertiles).

Sur les 3000 plantes utilisées en tant que parents femelles, 1250 se sont révélées mâles stériles et ont été effectivement utilisées.

16 plantes ms7/ms7 (mâles fertiles) ont cependant été conservées lors de l'épuration et autofécondées sous une enceinte hermétique constituée par un filet ou une toile ne laissant pas passer les insectes, afin d'être utilisées en tant que témoin d'autofécondation.

Les insectes pollinisateurs utilisés sont les mêmes que lors de l'essai 1.

Les apports en insectes pollinisateurs ont été réalisés à fréquence hebdomadaire durant les semaines 25 à 29 à raison de 1100 mouches par m² en moyenne par apport.

Parallèlement, 10 plantes mâles fertiles (parents mâles) et de 16 plantes ms7/ms7 fertiles conservées lors de l'étape d'épuration ont été autofécondées sous une enceinte hermétique constituée par un filet ou une toile ne laissant pas passer les insectes, afin de servir de témoins d'autofécondation.

La récolte des plantes mâles stériles (parents 'femelle') ainsi que de 10 plantes mâles fertiles (parents mâles) et de 16 plantes ms7/ms7 fertiles conservées lors de l'étape d'épuration utilisées comme témoins d'autofécondation a été réalisée en semaine 33.

Les plantes ont été séchées, battues, puis les graines ont été passées sur une colonne à air pulsé pour parfaire le nettoyage et éliminer les déchets légers résiduels et calibrées en longueur et en largeur.

Un échantillon des graines obtenues a été semé et la résistance des plantes obtenues au Bl24 a été testée selon un protocole officiel du GEVES (Groupe d'Etude et de contrôle des Variétés et des Semences). Les plantes se sont avérées résistantes au Bl24.

### Résultats des essais 1 et 2

Les résultats des essais 1 et 2 sont présentés dans les tableaux ci-dessous.

**Tableau 1 _{:} Comparaison des masses de graines récoltées lors des essais 1 et 2**

| | **Plantes** | **Masse récoltée (g)** | **Poids (g/plante)** |
|---|---|---|---|
| **Essai 1** | Témoin d'autofécondation (91 plantes parents « mâles ») | 2000 | 22 |
| | F1 MS7 (217 plantes parents « femelles » pollinisées par les mouches) | 730 | 3,36 |
| | Témoin d'autofécondation 1 (10 plantes parents « mâles ») | 83 | 8,3 |
| | Témoin d'autofécondation 2 (16 plantes parents « femelles » ms7/ms7 mâles fertiles) | 288 | 18 |
| | F1 MS7 (1170 plantes parents « femelles » pollinisées par les mouches) | 7730 | 6,61 |

**Tableau 2 : Comparaison des calibrages des graines hybrides et des graines issues des témoins d'autofécondation produits lors des essais 1 et 2**

| | **Plantes** | **calibre** | **Poids en g après nettoyage** | **%** | **% utile** | **% rebus** |
|---|---|---|---|---|---|---|
| **Essai 1** | Témoin d'autofécondation (91 plantes parents « mâles ») | >3,5-4,5< | 1774 | 89,10 | **89** | 10 |
| | | >4,5 | 200 | 10,05 | | |
| | | <3,5 | 17 | 0,85 | | |
| | | total | 1991 | | | |
| | F1 Ms7 (217 plantes parents « femelles » pollinisées par les mouches) | >3,5-4,5< | 620 | 93,83 | **94** | 6 |
| | | >4, 5 | 28,5 | 4,31 | | |
| | | <3,5 | 12,3 | 1,86 | | |
| | | total | 660,8 | | | |
| **Essai 2** | Témoin d'autofécondation 1 (10 plantes parents « mâles ») | >3,5-4,5< | 67,5 | 88,12 | **88** | 12 |
| | | >4,5 | 7,6 | 9,92 | | |
| | | <3,5 | 1,5 | 1,96 | | |
| | | total | 76,6 | | | |
| | Témoin d'autofécondation 2 (16 plantes parents « femelles » ms7/ms7 mâles fertiles) | >3,5-4,5< | 228,3 | 81,48 | **81** | 19 |
| | | >4,5 | 50,3 | 17,95 | | |
| | | <3,5 | 1,6 | 0,57 | | |
| | | total | 280, 2 | | | |
| | F1 Ms7 (1170 plantes parents « femelles » pollinisées par les mouches | >3,5-4,5< | 7730 | 98,85 | **99** | 1 |
| | | >4, 5 | 0 | 0 | | |
| | | <3,5 | 90 | 1,15 | | |
| | | total | 7820 | | | |

**Tableau 3 _{:} Comparaison des MMS (masses de mille semences) des graines hybrides et des graines issues des témoins d'autofécondation produits lors des essais 1 et 2**

| | **Plantes** | **mesure MMS 1 (g)** | **mesure MMS 1 (g)** | **mesure MMS 1 (g)** | **MMS moyenne (g)** |
|---|---|---|---|---|---|
| **Essai 1** | Témoin d'autofécondation (91 plantes parents « mâles ») | 0,9569 | 0,9603 | 0,954 | **0,9571** |
| | F1 Ms7 (217 plantes parents « femelles » pollinisées par les mouches) | 1,3609 | 1,3587 | 1,3484 | **1,356** |
| **Essai 2** | Témoin d'autofécondation 1 (10 plantes parents « mâles ») | 1,0784 | 1,0721 | 1,0745 | **1,075** |
| | Témoin d'autofécondation 2 (16 plantes parents « femelles » ms7/ms7 mâles fertiles) | 1,1144 | 1,1182 | 1,112 | **1,1149** |
| | F1 Ms7 (1170 plantes parents « femelles » pollinisées par les mouches | 1,4039 | 1,4036 | 1,4065 | **1,4047** |

### Discussion

Au cours du premier essai, 730 grammes de graines ont été obtenus à partir des 217 plantes *Lactuca sativa* mâles stériles (parent femelles) soit un rendement de 3,36 grammes par plante, correspondant à un ratio de 15,30% du témoin auto-fécondé (parent mâle).

Au cours du second essai, 7,730 kg de graines ont ainsi été obtenus à partir de 1170 plantes *Lactua sativa* mâles stériles (parent femelle), soit un rendement de 6,60 grammes par plante, correspondant à un ratio de 50% de la moyenne des deux témoins auto-fécondés (parents « mâles » et parents « femelles » mâles fertiles ms/ms conservées lors de l'épuration et fécondées sous une enceinte hermétique aux insectes).

Lors du deuxième essai, le taux de semences utiles après calibrage des hybrides F1 est supérieur de 10% au témoin auto-fécondé.

Lors des deux essais, la MMS (masse de mille semences) de l'hybride F1 est supérieure de 20 à 30% du témoin auto-fécondé.

Les plantes obtenues à partir des graines obtenues selon le second essai sont bien résistantes au Bl24.

### Essais 3 à 8

Six essais similaires ont été lancés en parallèle avec différents types de laitues beurres et batavia dans des cages insect proof de 3.3m x 1.1m comptant chacune 8 plantes Lactuca sativa utilisées en tant que parents « femelles » (à 50% mâles stériles, issues d'un rétrocroisement entre des plantes Lactuca sativa porteuses du gène Ms7 et des plantes Lactuca sativa fertiles), et 4 plantes Lactuca sativa mâles fertiles utilisées en tant que parents « mâles », placées dans une serre ventilée.

Les semis ont été effectués en semaine 22, les plantations en semaine 27, le tri entre les plantes « femelles » mâles stériles et mâles fertiles au début de la floraison en semaine 31, les apports en mouches en semaines 32, 33 et 34 à raison d'un apport par semaine pendant trois semaines, soit environ 100 mouches/m², et la récolte en semaine 37.

### Résultats des essais 3 à 8

- Cage 1: CHARLIN(68/12624)*CHARLIN (beurre)
   1 plante femelle : 9.20 gr
   1 plante mâle : 28.30 gr
- **Cage 2:** CHARLIN(BC /77)*CHARLIN (beurre)
   1 plante femelle : 5.45 gr
   1 plante mâle : 19.40 gr
- **Cage 3** : BRA 68/12588 (laitue pommée d'abri) * BRA 68/12588
   6 plantes femelle
      plante 1 : 7.00 gr
      plante 2 : 2.00 gr
      plante 3 : 5.20 gr
      plante 4 : 3.40 gr
      plante 5 : 9.60 gr
      plante 6 : 8.40 gr
      moyenne : 5.9 gr/plante
   1 plante mâle : 34.40 gr
- **Cage 4** : BRA 68/12588 * BRA 68/12588
   4 plantes femelles :
      plante 1 : 11.95 gr
      plante 2 : 7.15 gr
      plante 3 : 15.90 gr
      plante 4 : 11.90 gr
      moyenne 11.25 gr/plante
   1 plante mâle : 29.00 gr
- Cage 5 : BVA 68/12553 (laitue batavia d'abri)* BVA 68/12553
   5 plantes femelles :
      plante 1 : 3.50 gr
      plante 2 : 1.30 gr
      plante 3 : 2.20 gr
      plante 4 : 1.70 gr
      plante 5 : 4.20 gr
      moyenne 3.30 gr/plante
   1 plante mâle : 3.60 gr
- **Cage 6** : BVA 68/12553 * BVA 68/12553
   3 plantes femelles :
      plante 1 : 4.60 gr
      plante 2 : 3.20 gr
      plante 3 : 3.70 gr
      moyenne : 3.80 gr/plante
   1 plante mâle : 23.40 gr

### Discussion

Les moyennes de production des parents « femelles » s'étalent de 3.3g à 11.2 g/plante, la typologie batavia **(BVA 68/12553)** ayant un rendement nettement inférieur (environ 3.5 g/plante) par rapport à une typologie beurre (Charlin, **BRA 68/12588)** environ 8.5 g/plante, ce qui peut laisser à penser que l'on a une forte implication de la typologie sur le rendement finale.

### Essai 9

Un nouvel essai de production a été mené sous des petites cages hermétiques aux insectes de 0.40m de diamètre, placées en serre ventilée en pleine terre.

Les semis ont été effectués en semaine 22, les rempotages en semaine 25, le tri entre les plantes « femelles » mâles stériles et mâles fertiles au début de la floraison en semaine 30, les plantations en semaine 31, les apports en mouches en semaines 32, 33 et 34 à raison d'un apport par semaine pendant 3 semaines, soit environ 100 mouches/m², et la récolte en semaine 35.

### Résultats

Les résultats obtenus sont synthétisés dans les tableaux 4 et 5 ci-dessous.

**Tableau 4 : production de graines par les parents « mâles » et « femelles » Charlin (beurre d'abri)**

| **Parent femelle Charlin** | **Poids en gr** | **Parent mâle Charlin** | **Poids en gr** |
|---|---|---|---|
| 1 | 3,3 | 1 | 1,65 |
| 2 | 0,76 | 2 | 1,7 |
| 3 | 0,44 | 3 | 3 |
| 4 | 1,1 | 4 | 1,18 |
| 5 | 4,18 | 5 | 1,9 |
| 6 | 2,7 | 6 | 2,95 |
| 7 | 1,72 | 7 | 1,9 |
| 8 | 1,78 | 8 | 2,3 |
| 9 | 1,92 | | |
| **Moyenne** | **1,99** | **moyenne** | **2,0725** |

**Tableau 5 : production de graines par les parents « mâles » et « femelles » BVA 68/12553 (batavia)**

| **Parent femelle BVA 68/12553 (batavia)** | **Poids en gr** | **Parent mâle BVA 68/12553 (batavia)** | **Poids en gr** |
|---|---|---|---|
| 1 | 1,1 | 1 | 1,45 |
| 2 | 1,17 | 2 | 0,95 |
| 3 | 1,2 | 3 | 2 |
| 4 | 1,48 | 4 | 1,6 |
| 5 | 0,66 | 5 | 1,27 |
| 6 | 0,69 | 6 | 3 |
| 7 | 0,55 | 7 | 1,8 |
| 8 | 1,22 | 8 | 1,9 |
| 9 | 0,75 | 9 | 1,26 |
| 10 | 1,3 | 10 | 2,3 |
| 11 | 1,57 | 11 | 1,15 |
| 12 | 0,52 | 12 | 1,4 |
| 13 | 0,55 | | |
| **Moyenne** | **0,98** | **moyenne** | **1,67** |

### Discussion

Les résultats sont satisfaisants dans la mesure où la production moyenne obtenue pour la laitue batavia **(BVA 68/12553)** est d'environ 1gr/plante contre 1.7 gr/plante pour le témoin autofecondé, et la production moyenne obtenue pour la laitue beurre d'abri (Charlin) est d'environ 1gr/plante, au même niveau que le témoin autofécondé.

### Essai 10 : Cumul Dm3/Dm18 par combinaison hybride

Des Laitues hybrides F1 cumulant deux gènes dominants Dm3 et Dm18 de résistance au *Brémia lactucae* respectivement race 24 (Bl24) et race 23 (Bl23), appartenant au cluster 2 et situés sur le même locus ont été obtenues selon la méthode décrite dans les essais décrits plus haut.

Les plantes utilisées en tant que parents « femelles » (06/30443 : MS7/21NACRExDEVONIAxCAMBRIA) sont porteuses des gènes de résistance Dm18 et R38. Les plantes utilisées en tant que parents « mâles » sont des beurres d'abri porteuses du gène de résistance Dm3 (BC:06/30443 * Rex ou BC:06/30443 * Melina).

Les cinq croisements suivants ont été réalisés :
- 06/30443/01 (MS7/21xNACRExDEVONIAxCAMBRIA-01) **Dm18/R38** * Rex **Dm3**
- 06/30443/10 (MS7/21xNACRExDEVONIAxCAMBRIA-10**) Dm18/R38** * Rex **Dm3**
- 06/30443/02 (MS7/21xNACRExDEVONIAxCAMBRIA-02) **Dm18/R38** * Melina **Dm3**
- 06/30443/03 (MS7/21xNACRExDEVONIAxCAMBRIA-03) **Dm18/R38** * Melina **Dm3**
- 06/30443/04 (MS7/21xNACRExDEVONIAxCAMBRIA-04) **Dm18/R38** * Melina **Dm3**

Les graines issues de ces cinq croisements ont été semées et des tests d'inoculation sur disques foliaires et de marquage moléculaire ont été réalisés sur les plantes F1, ainsi que sur les parents « mâles » (Rex/Melina) et « femelles ».

### I - Tests d'inoculation sur disques foliaires

*Matériel :* Le matériel végétal est constitué de disques foliaires de 1,5 cm de diamètre à raison de 5 disques par plante et par race, de 10 plantes par origine et de deux races *Bremia lactucae* (Bl24 qui attaque le cumul Dm18/R38 et auquel Dm3 confère une résistance, et B123 qui attaque le cumul Dm3/R38 et auquel Dm18 confère une résistance). Ainsi 80 plantes ont été testées au total sur deux races. *Technique* : Les disques foliaires sont inoculés par pulvérisation de sporocystes avec les races de Brémia Bl23 et Bl24 séparément.
1/ Prélèvement des disques foliaires : cinq disques foliaires par plantes sont prélevés et déposés sur une double épaisseur de papier buvard humidifiée. Chaque boîte contient 6 lignes de disques à tester et une lignée de témoin sensible.
2/Inoculation : la quantité d'inoculum nécessaire pour inoculer 6 boîtes de disques foliaires est de 11 ml. Une solution mère de Tween 80 est préparée à raison de 4 gouttes de Tween 80 ajoutés à 100 ml d'eau déminéralisée. La quantité de solution d'extraction pour le volume d'inoculum est de 5 ml de solution mère de Tween 80 pour 100 ml d'inoculum. L'inoculum est réalisé en détachant les sporocystes dans de l'eau du robinet additionnée de 5 % de solution mère de tween 80. Les sporocystes sont prélevés à raison d'1 plante/ml d'inoculum, pour obtenir une concentration en sporocystes proche de 10⁸ spores/ml. Une filtration est réalisée en passant la solution sur une chiffonnette dans un bêcher de 250 ml.
3/Pulvérisation : l'inoculation se déroule le même jour que le prélèvement des disques foliaires. Le volume de solution d'inoculum est mesuré puis les boites étalées sont inoculées. L'inoculum est pulvérisé de façon homogène en fines gouttelettes sur l'ensemble de la surface de chaque disque. Les boites sont refermées hermétiquement et incubées en module à 15°C, 14 h de jour, 10 h de nuit pendant 7 jours.

Résultats : les résultats des inoculations sont présentés dans les tableaux 6 à 13 ci-dessous (S=sensible ; R=résistant). Les hybrides issus des différents croisements sont résistants aux deux races Bl23 et Bl24, indiquant la présence des deux gènes Dm18 et Dm3 du cluster 2.

**Tableau 6 _{:} Plantes « femelles » 30443 (Dm18/R38) : MS7/21xNACRExDEVONIAxCAMBRIA : Résistantes Bl23 et sensibles Bl24.**

| **GENOTYPE** | **PLANTE** | **B123** | **B124** |
|---|---|---|---|
| 06/30443 | 2 | R | S |
| 06/30443 | 3 | R | S |
| 06/30443 | 4 | R | S |
| 06/30443 | 5 | R | S |
| 06/30443 | 6 | R | S |
| 06/30443 | 7 | R | S |
| 06/30443 | 8 | R | S |
| 06/30443 | 9 | R | S |
| 06/30443 | 10 | R | S |

**Tableau 7 : Plantes « mâles » Melina (Dm3) : sensibles Bl23 et résistantes B124 (test sur 10 plantes)**

| **GENOTYPE** | **PLANTE** | **B123** | **B124** |
|---|---|---|---|
| MELINA | 1 | S | R |
| MELINA | 2 | S | R |
| MELINA | 3 | S | R |
| MELINA | 4 | S | R |
| MELINA | 5 | S | R |
| MELINA | 6 | S | R |
| MELINA | 7 | S | R |
| MELINA | 8 | S | R |
| MELINA | 9 | S | R |
| MELINA | 10 | S | R |

**Tableau 8 : Croisement 06/30443/02 * Melina (Dm18/R38*Dm3) = MS7/21xNACRExDEVONIAxCAMBRIA-02*MELINA.**

| **GENOTYPE** | **PLANTE** | **B123** | **B124** |
|---|---|---|---|
| 06/30443/02*MELINA | 2 | R | R |
| 06/30443/02*MELINA | 3 | R | R |
| 06/30443/02*MELINA | 4 | R | R |
| 06/30443/02*MELINA | 5 | R | R |
| 06/30443/02*MELINA | 6 | R | R |
| 06/30443/02*MELINA | 7 | R | R |
| 06/30443/02*MELINA | 8 | R | R |
| 06/30443/02*MELINA | 9 | R | R |
| 06/30443/02*MELINA | 10 | R | R |

**Tableau 9 : Croisement 06/30443/03 * Melina (Dm18/R38*Dm3) = MS7/21xNACRExDEVONIAxCAMBRIA-03*MELINA.**

| **GENOTYPE** | **PLANTE** | **B123** | **B124** |
|---|---|---|---|
| 06/30443/03*MELINA | 2 | R | R |
| 06/30443/03*MELINA | 3 | R | R |
| 06/30443/03*MELINA | 4 | R | R |
| 06/30443/03*MELINA | 5 | R | R |
| 06/30443/03*MELINA | 6 | R | R |
| 06/30443/03*MELINA | 7 | R | R |
| 06/30443/03*MELINA | 8 | R | R |
| 06/30443/03*MELINA | 9 | R | R |
| 06/30443/03*MELINA | 10 | R | R |

**Tableau 10 : Croisement 06/30443/04 * Melina (Dm18/R38*Dm3) = MS7/21xNACRExDEVONIAxCAMBRIA-03*MELINA.**

| **GENOTYPE** | **PLANTE** | **B123** | **B124** |
|---|---|---|---|
| 06/30443/04*MELINA | 2 | R | R |
| 06/30443/04*MELINA | 3 | R | R |
| 06/30443/04*MELINA | 4 | R | R |
| 06/30443/04*MELINA | 5 | R | R |
| 06/30443/04*MELINA | 6 | R | R |
| 06/30443/04*MELINA | 7 | R | R |
| 06/30443/04*MELINA | 8 | R | R |
| 06/30443/04*MELINA | 9 | R | R |
| 06/30443/04*MELINA | 10 | R | R |

**Tableau 11 : plantes « mâles » Rex (Dm3) : sensibles Bl23 et résistante Bl24 (test sur 10 plantes)**

| **GENOTYPE** | **PLANTE** | **B123** | **B124** |
|---|---|---|---|
| REX/1 | 1 | S | R |
| REX/2 | 2 | S | R |
| REX/3 | 3 | S | R |
| REX/4 | 4 | S | R |
| REX/5 | 5 | S | R |
| REX/6 | 6 | S | R |
| REX/7 | 7 | S | R |
| REX/8 | 8 | S | R |
| REX/9 | 9 | S | R |
| REX/10 | 10 | S | R |

**Tableau 12 : Croisement 06/30443/01*Rex (Dm18/R38*Dm3) = MS7/21xNACRExDEVONIAxCAMBRIA-01*REX.**

| **GENOTYPE** | **PLANTE** | **B123** | **B124** |
|---|---|---|---|
| 06/30443/01*REX | 2 | R | R |
| 06/30443/01*REX | 3 | R | R |
| 06/30443/01*REX | 4 | R | R |
| 06/30443/01*REX | 5 | R | R |
| 06/30443/01*REX | 6 | R | R |
| 06/30443/01*REX | 7 | R | R |
| 06/30443/01*REX | 8 | R | R |
| 06/30443/01*REX | 9 | R | R |
| 06/30443/01*REX | 10 | R | R |

**Tableau 13 : Croisement 06/30443/10*Rex (Dm18/R38*Dm3) = MS7/21xNACRExDEVONIAxCAMBRIA-10*REX.**

| **GENOTYPE** | **PLANTE** | **B123** | **B124** |
|---|---|---|---|
| 06/30443/02*REX | 2 | R | R |
| 06/30443/02*REX | 3 | R | R |
| 06/30443/02*REX | 4 | R | R |
| 06/30443/02*REX | 5 | R | R |
| 06/30443/02*REX | 6 | R | R |
| 06/30443/02*REX | 7 | R | R |
| 06/30443/02*REX | 8 | R | R |
| 06/30443/02*REX | 9 | R | R |
| 06/30443/02*REX | 10 | R | R |

### II - Marquage moléculaire

L'utilisation de 2 marqueurs moléculaires permet de mettre en évidence le cumul de deux gènes très liés (situés sur le même locus), dominants, de résistance à deux souches de *Bremia lactucae* chez *Lactuca sativa.*

### Méthode :

1/ Matériel végétal : le matériel végétal est constitué de disques foliaires de 1,5 cm de diamètre à raison d'un disque par plante. L'ADN de 5 à 10 plantes par génotype (soit 50 plantes au total) a été obtenu à partir d'une extraction CTAB/Chloroforme suivie d'une remise en suspension dans une solution de TE 0,1X à une concentration de 5ng/µl.
2/ Génotypage : les marqueurs SCW09 et B1 décrits respectivement par Maisonneuve et al. **[10]** et Kuang H. et al. **[11]** ont été utilisés pour génotyper les 50 plantes (tableau 14). Les conditions de PCR sont décrites dans le tableau 15.

Les cycles PCR de SCW09 et B1 sont respectivement :
- 94°C/30s - 94°C/1mn, 60°C/1mn, 72°C/2min; 40 cycles - 72°C/5min - 4°C ;
- 94°C/30s - 94°C/lmn, 63°C/1mn, 72°C/2min; 40 cycles - 72°C/5min - 8°C.

Le marqueur SCW09 a subit une digestion enzymatique avec l'enzyme Taq I avant l'étape de migration (tampon 1X, BSA 1X et Taq I 1U). L'électrophorèse a été effectuée sur gel d'agarose à 2% (SCW09) ou 1,5% (B1) dans un tampon de TBE 1X pendant 1h (B1) à 1h30 (SCW09) avec un voltage constant de 220 V. La révélation des profils électrophorétiques a été faite par coloration au bromure d'éthidium (BET) sous lampe UV.

**Tableau 14 : Caractéristiques des marqueurs SCW09 et B1**

| **Locus** | **Test** | **Type** | **Primer** | **Séquence** | **Tm** | **GC%** | **Polymorphisme** |
|---|---|---|---|---|---|---|---|
| SCW09 | Dm18 | CAPS | SCW09-A | GTGACCGAGTAGTCTTAACCTAGT (SEQ ID N°3) | 61,0 | 46% | Co-dominant |
| | | | SCW09-B | GTGACCGAGTGTAACAACGTAAAT (SEQ ID N°4) | 59,3 | 42% | |
| B1 | Dm3 | SCAR | B1F | GAGAATAGAGTCTTGTGATGGCA (SEQ ID N°5) | 58,9 | 43% | Dominant |
| | | | B1R | CCCGTAAGACATGGAAGTTCTCT (SEQ ID N°6) | 60,6 | 48% | |

**Tableau 15 : Conditions PCR des marqueurs SCW09 et B1**

| **Produits chimiques** | **SCW09** | **B1** |
|---|---|---|
| H20 | qsp 20 µl | qsp 20 µl |
| Tampon | 1X | 1X |
| MgCl2 | 2,5mM | 1,5 mM |
| Dntp | 0,2mM | 200µM |
| Amorce forward | 0,04 µM | 100 nM |
| Amorce reverse | 0,04 µM | 100 nM |
| Taq Polymerase Cetus | 1u | 1U |
| DNA | 5 µl | 5 µl |

### Résultats

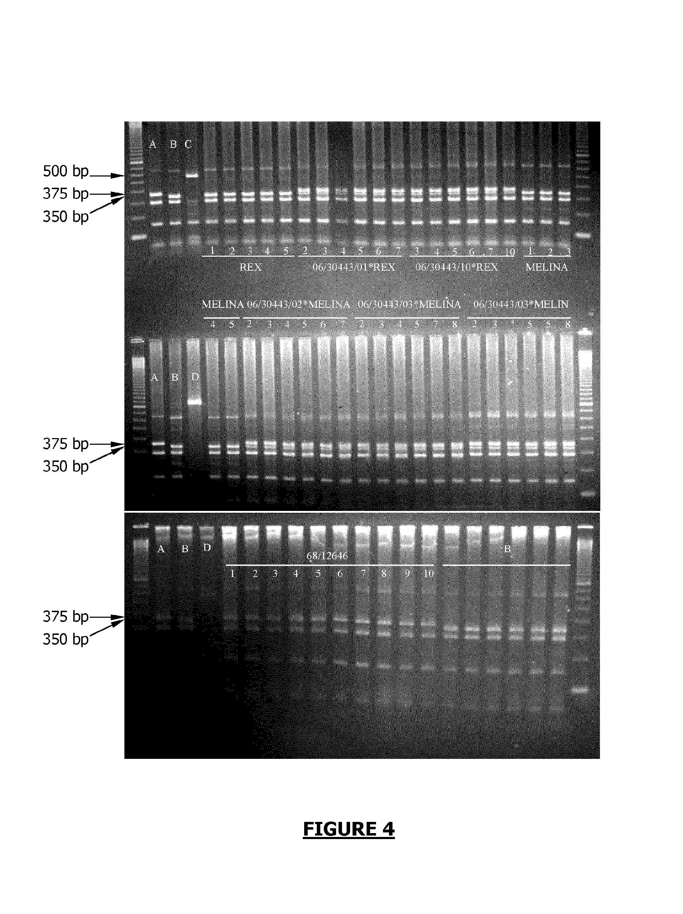

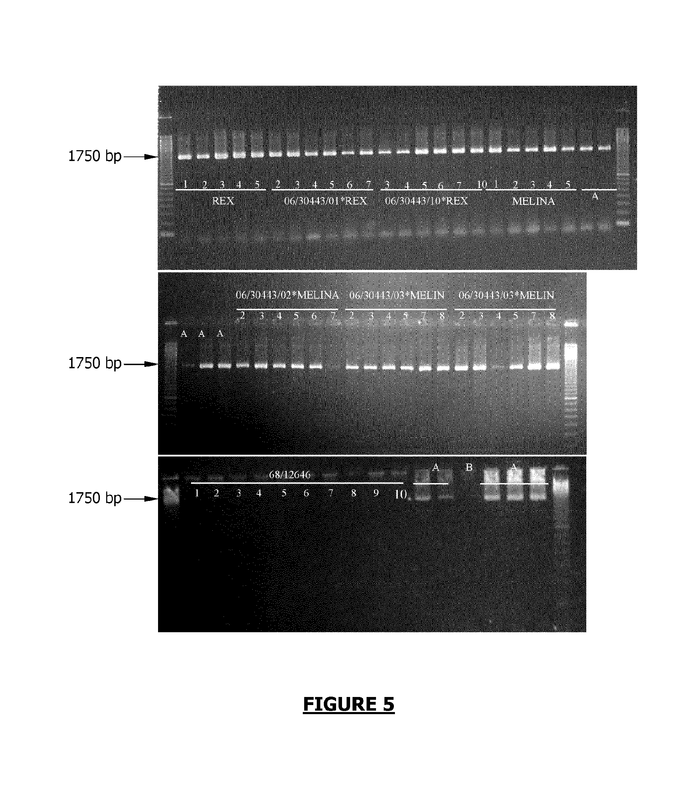

Les résultats sont présentés dans le tableau 16 et les figures 4 et 5. Les génotypes REX et MELINA ont des profils Dm18-/Dm18- et Dm3+. Le génotype 06/30443 a un profil Dm18+/Dm18+ et Dm3-. L'ensemble des croisements avec l'un ou l'autre des parents (REX ou MELINA) ont des profils Dm18+/Dm18- et Dm3+ c'est à dire résistants pour Dm18 et Dm3.

**Tableau 16 : Résultats de génotypage avec les marqueurs SCW09 et B1 (S = sensible ; R = résistant)**

| **N° GENE** | **GENOTYPE** | **PLANTE** | **B123 (Dm18)** | **B124 (Dm3)** | **Dm3 (B1)** | **DM18 (SCW09)** |
|---|---|---|---|---|---|---|
| G02766 | REX | 1 | S | R | Dm3+ | Dm18-/Dm18- |
| G02767 | REX | 2 | S | R | Dm3+ | Dm18-/Dm18- |
| G02768 | REX | 3 | S | R | Dm3+ | Dm18-/Dm18- |
| G02769 | REX | 4 | S | R | Dm3+ | Dm18-/Dm18- |
| G02770 | REX | 5 | S | R | Dm3+ | Dm18-/Dm18- |
| G02771 | 06/30443/01*REX | 2 | R | R | Dm3+ | Dm18+/Dm18- |
| G02772 | 06/30443/01*REX | 3 | R | R | Dm3+ | Dm18+/Dm18- |
| G02773 | 06/30443/01*REX | 4 | R | R | Dm3+ | Dm18+/Dm18- |
| G02774 | 06/30443/01*REX | 5 | R | R | Dm3+ | Dm18+/Dm18- |
| G02775 | 06/30443/01*REX | 6 | R | R | Dm3+ | Dm18+/Dm18- |
| G02776 | 06/30443/01*REX | 7 | R | R | Dm3+ | Dm18+/Dm18- |
| G02777 | 06/30443/10*REX | 3 | R | R | Dm3+ | Dm18+/Dm18- |
| G02778 | 06/30443/10*REX | 4 | R | R | Dm3+ | Dm18+/Dm18- |
| G02779 | 06/30443/10*REX | 5 | R | R | Dm3+ | Dm18+/Dm18- |
| G02780 | 06/30443/10*REX | 6 | R | R | Dm3+ | Dm18+/Dm18- |
| G02781 | 06/30443/10*REX | 7 | R | R | Dm3+ | Dm18+/Dm18- |
| G02782 | 06/30443/10*REX | 10 | R | R | Dm3+ | Dm18+/Dm18- |
| G02783 | MELINA | 1 | S | R | Dm3+ | Dm18-/Dm18- |
| G02784 | MELINA | 2 | S | R | Dm3+ | Dm18-/Dm18- |
| G02785 | MELINA | 3 | S | R | Dm3+ | Dm18-/Dm18- |
| G02786 | MELINA | 4 | S | R | Dm3+ | Dm18-/Dm18- |
| G02787 | MELINA | 5 | S | R | Dm3+ | Dm18-/Dm18- |
| G02788 | 06/30443/02*MELINA | 2 | R | R | Dm3+ | Dm18+/Dm18- |
| G02789 | 06/30443/02*MELINA | 3 | R | R | Dm3+ | Dm18+/Dm18- |
| G02790 | 06/30443/02*MELINA | 4 | R | R | Dm3+ | Dm18+/Dm18- |
| G02791 | 06/30443/02*MELINA | 5 | R | R | Dm3+ | Dm18+/Dm18- |
| G02792 | 06/30443/02*MELINA | 6 | R | R | Dm3+ | Dm18+/Dm18- |
| G02793 | 06/30443/02*MELINA | 7 | R | R | Dm3+ | Dm18+/Dm18- |
| G02794 | 06/30443/03*MELINA | 2 | R | R | Dm3+ | Dm18+/Dm18- |
| G02795 | 06/30443/03*MELINA | 3 | R | R | Dm3+ | Dm18+/Dm18- |
| G02796 | 06/30443/03*MELINA | 4 | R | R | Dm3+ | Dm18+/Dm18- |
| G02797 | 06/30443/03*MELINA | 5 | R | R | Dm3+ | Dm18+/Dm18- |
| G02798 | 06/30443/03*MELINA | 7 | R | R | Dm3+ | Dm18+/Dm18- |
| G02799 | 06/30443/03*MELINA | 8 | R | R | Dm3+ | Dm18+/Dm18- |
| G02800 | 06/30443/04*MELINA | 2 | R | R | Dm3+ | Dm18+/Dm18- |
| G02801 | 06/30443/04*MELINA | 3 | R | R | Dm3+ | Dm18+/Dm18- |
| G02802 | 06/30443/04*MELINA | 4 | R | R | Dm3+ | Dm18+/Dm18- |
| G02803 | 06/30443/04*MELINA | 5 | R | R | Dm3+ | Dm18+/Dm18- |
| G02804 | 06/30443/04*MELINA | 7 | R | R | Dm3+ | Dm18+/Dm18- |
| G02805 | 06/30443/04*MELINA | 8 | R | R | Dm3+ | Dm18+/Dm18- |
| G02806 | 06/30443 | 1 | R | S | Dm3- | Dm18+/Dm18+ |
| G02807 | 06/30443 | 2 | R | S | Dm3- | Dm18+/Dm18+ |
| G02808 | 06/30443 | 3 | R | S | Dm3- | Dm18+/Dm18+ |
| G02809 | 06/30443 | 4 | R | S | Dm3- | Dm18+/Dm18+ |
| G02810 | 06/30443 | 5 | R | S | Dm3- | Dm18+/Dm18+ |
| G02811 | 06/30443 | 6 | R | S | Dm3- | Dm18+/Dm18+ |
| G02812 | 06/30443 | 7 | R | S | Dm3- | Dm18+/Dm18+ |
| G02813 | 06/30443 | 8 | R | S | Dm3- | Dm18+/Dm18+ |
| G02814 | 06/30443 | 9 | R | S | Dm3- | Dm18+/Dm18+ |
| G02815 | 06/30443 | 10 | R | S | Dm3- | Dm18+/Dm18+ |

### EXEMPLE 2 : IDENTIFICATION D'UN MARQUEUR MOLÉCULAIRE DU GENE Ms7.

La méthode d'obtention d'un marqueur moléculaire associé à la stérilité mâle Ms7 chez *Lactuca sativa* (laitue) décrite ci-après est donnée à titre indicatif.

### 1 - Construction de populations pour le développement d'un marqueur lié à la stérilité mâle nucléaire Ms7 chez la laitue (L. sativa).

Deux populations de typologies différentes de laitues présentant une stérilité mâle Ms7 ont été réalisées par rétro-croisement. Deux populations récurrentes de quatrième génération (BC4) de 200 plantes ainsi obtenues ont ensuite été phénotypées sur le caractère « absence de pollen viable » correspondant au phénotype mâle stérile. Deux groupes phénotypiques ont été identifiés comme mâle fertile (F) ou mâle stérile (S) selon un ratio de 1:1.

Ce ratio confirme bien la nature monogénique dominante de la stérilité mâle apportée par le parent hétérozygote au locus Ms7.

### 2 - Extraction de l'ADN des plantes de laitue.

L'extraction de l'ADN s'est faite suivant le protocole CTAB modifié (Tomas et al., 1989 **[20];** Doyle et al., 1990 **[15];** Edwards et al., 1991 **[16])** sur des jeunes feuilles fraîches.

Des disques foliaires frais obtenus à l'aide d'un tube de 1,5 ml sont broyés dans 500 µl de tampon d'extraction (tris-HCl 0,1M, NaCl 0,7M, EDTA 10mM, CTAB 1%, β-Mercaptoethanol 1%) . Le broyat est incubé pendant 1h à 65°C et mélangé 2 à 3 fois par retournement au cours de l'incubation.

200 µl d'une solution de chloroform:isoamyl alcohol 24:1 sont ensuite ajoutés et mélangés par retournement. Après centrifugation à 6000 rpm pendant 10 minutes à 20°C, 400 µl de surnageant sont récupérés et mélangés à 400 µl d'isopropanol.

Après 1h à -20°C, le mélange est centrifugé à 6000 rpm pendant 10 minutes à 4°C. Les tubes sont vidés. Le culot d'ADN fixé au fond du tube est mis à sécher à l'air pendant 12h.

L'ADN est remis en suspension dans 200 µl d'une solution de TE 0,1X (Tris-HCl 1 mM, EDTA 0,1 mM). La concentration finale est mesurée par dosage par Lambda DNA-Hind III Digest sur gel d'agarose 1%.

### 3 - Recherche d'un marqueur RAPD associé à la stérilité mâle Ms7 chez L. sativa par la méthode de BSA (Bulk Segregant Anaysis).

Dans le but d'identifier un marqueur moléculaire associé à la stérilité mâle Ms7, les RAPD® 10mer kits de Operon Technologies Inc. (Huntsville, AL 35805, USA) et la méthode de Bulk Segregant Analysis (Michelmore et al., 1991 **[17]** et Paran et al., 1991 **[18]**) ont été utilisés.

À partir du phénotypage des deux populations BC4 décrites précédemment, 2 échantillonnages de mélanges de 10 plantes ont été réalisés pour les deux groupes « mâle stérile » et « mâle fertile ». 1200 amorces Operon Biotechnologies Inc. (Huntsville, AL 35805, USA) de OPA-01 à OPBH-20 ont été testées par la technique de RAPD décrite par William et al., 1990 **[22]** et, Welsh et al., 1990 **[21]** sur 4 échantillons par population. Les amorces mettant en évidence une bande spécifique aux échantillons « mâle stérile » et présentant un profil d'électrophorèse de lecture facile ont été sélectionnées.

La réaction de PCR est réalisée dans un volume réactionnel total de 25 µl constitués de tampon PCR 1X, 3 mM de MgCl2, 200 µM de dNTPs, 400 nM d'amorce, 1 Unité d'ADN polymérase AmpliTaq (Perkin-Elmer cetus). La réaction de PCR consiste en plusieurs cycles décrits comme suit : 1 étape à 94°C pendant 30 s ; 45 cycles à 94°C pendant 1 min puis 35°C pendant 1min et enfin 72°C pendant 2 min et, une élongation finale réalisée à 72°C pendant 5 min, la réaction étant ensuite conservée à 4°C.

Les produits d'amplification sont séparés sur un gel d'agarose à 2% dans les conditions d'électrophorèse suivantes : tampon de migration TBE 1X (Tris-Borate-EDTA) à 190V pendant 2H15.

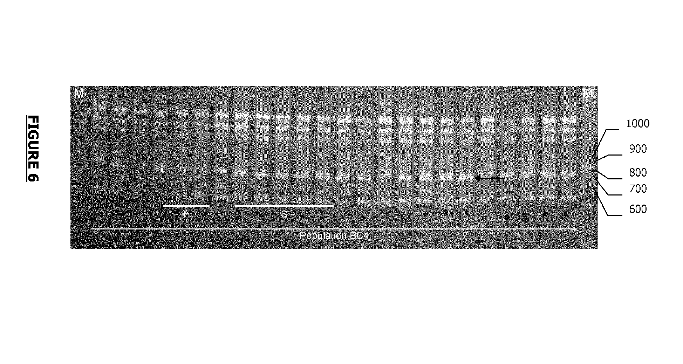

Afin de valider les amorces RAPD retenues, chacune des 200 plantes des deux populations BC4 a été testée individuellement pour l'amorce OPBA05 ayant comme séquence « 5' TGCGTTCCAC 3' » (SEQ ID N°1) et à laquelle correspond un marqueur de 675 pb, BA05-675 (SEQ ID N°2), qui co-ségrège avec Ms7. Le profil électophorétique obtenu pour ce marqueur est montré à la figure 6.

Une validation de ce marqueur sur différentes typologies de laitues (batavia, beurre, iceberg, romaine, feuille de chêne et lollo rossa) a été réalisée sur environ 25 plantes de 12 populations. Les conditions de réalisation du test sont identiques à celles décrites précédemment pour les étapes d'extraction, d'amplification et d'électrophorèse.

Le calcul de la sensibilité et de la spécificité du marqueur RAPD BA05-675 montre des valeurs différentes, suivant les typologies, et des valeurs moyennes respectives de 96% et 94% (voir tableaux 17 et 18).

**Tableau 17 : Détail du calcul des valeurs prédictives, sensibilité et spécificité des marqueurs moléculaires retenus.**

| | **PHENOTYPE** | | |
|---|---|---|---|
| | **Mâle Stérile** | **Mâle Fertile** | |
| **MARQUEUR 1 (présent [1] ou absent 0 [0])** | Nombre de vrais positifs (#VP) | Nombre de faux positifs (#FP) | **Valeur prédictive positive = #VP / (#VP + #FP)** |
| | Nombre de faux négatifs (#FN) | Nombre de vrais négatifs (#VN) | **Valeur prédictive négative = #VN / (#VN + #FN)** |
| | **SENSIBILITE = #VP / (#VP + #FN)** | **SPECIFICTE = #VN / (#VN + #FP)** | |

**Tableau 18 : Résultats par typologie de laitue et valeur moyenne du calcul de la sensibilité (S*) et de la spécificité (F**) du marqueur moléculaire BA05-675 associé à Ms7.**

| ***POP.*** | **BA05-675** | |
|---|---|---|
| | **S*** | **F**** |
| BATAVIA | 100% | 100% |
| LOLLO ROSSA | 100% | 100% |
| BEURRE | 100% | 91% |
| ICEBERG | 95% | 100% |
| ROMAINE | 100% | 71,4% |
| FEUILLE DE CHENE | 82% | 100% |
| ***Moyenne*** | **96%** | **94%** |

### 4 - Cartographie génétique du marqueur BA05-675 associé à la stérilité mâle Ms7 chez l'espèce Lactuca sativa.

À partir des 200 plantes d'une population BC4 en disjonction pour Ms7 décrite ci-dessus, la cartographie génétique (William et al., 1993 **[23])** du marqueur BA05-675 associé à Ms7 a été réalisée à l'aide des programmes JoinMap®4 (Stam et al., 1996 **[19])** et Carte Blanche© (Keygene N.P., P.O. Box 216, 6700 AE Wageningen, The Netherlands). Le test statistique du X² a permis de vérifier l'hypothèse nulle d'une ségrégation de type mendélienne de rapport 1:1 (test non significatif à p>0,05) pour le marqueur dominant BA05-675 (voir tableau 19). Un test de liaison avec un rapport de vraisemblance (ou LOD score) supérieur ou égale à 3,0 a permis de cartographier ce marqueur à 1,8 cM de Ms7 (figure 6).

**Tableau 19 : Rapports de ségrégation marqueur moléculaire BA05-675 associé à Ms7 et résultats du test de X² à 1 ddl selon l'hypothèse nulle d'une ségrégation de type mendélienne de rapport 1:1. (F : nombre de plantes mâles fertiles ; S : nombre de plantes mâles stériles)**

| **Locus** | **F:S** | **X2 (1:1)** |
|---|---|---|
| BA05-675 | 103:95 | 0,25 |

### 5 - Clonage et séquençage du marqueur moléculaire RAPD BA05-675 associé à la stérilité mâle chez la laitue, Lactuca sativa.

À partir du profil électrophorétique sur gel d'agarose, le fragment d'ADN de 675pb a été isolé et remis en suspension dans un tampon TE (10 mM Tris-Cl, 1 mM EDTA) puis ré-amplifié suivant les conditions PCR décrites ci-dessus. Le fragment isolé ré-amplifié a été cloné à l'aide du kit commercial pCR®4-TOPO® (Invitrogen, Carlsbad, California 92008, USA). Après vérification du clonage, des extractions d'ADN (système Midiprep) ont été effectuées à l'aide du Kit de purification sur colonne Promega (Madison, WI, USA).

Les clones purifiés concentrés à 75ng/µl ont été séquencés par Cogenics (38944 Meylan, France) . La séquence du marqueur BA05-675 décrite en figure 8 a été ainsi obtenue (SEQ ID N°2) .

### 6- Résultats

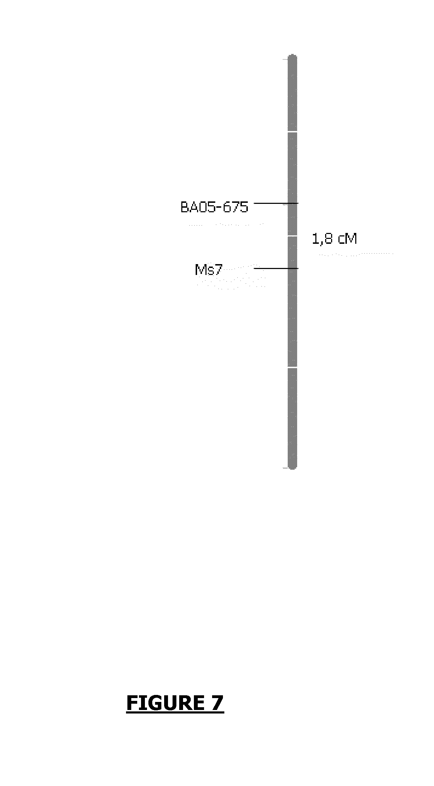

L'étude menée par les inventeurs a permis la définition d'un marqueur RAPD, BA05-675 (SEQ ID N°2), dont la séquence est présentée en figure 8, distant de 1,8 cM de Ms7 (figure 7) et présentant une sensibilité et une spécificité moyennes de respectivement 96% et 94% indépendamment de la typologie de laitue (tableau 18). L'évaluation de ce marqueur sur les plantes individuelles des 2 populations BC4 a permis d'estimer que la valeur prédictive (Altman 1994a **[13])** d'identification des plantes mâles stériles est en moyenne de près de 97%.

### Bibliographie

**1.** Goubara & Takasaki (2003) Flower visitors of lettuce under field and enclosure conditions, Appl. Entomo. Zool. 38(4) : 571-581.
**2.** Goubara & Takasaki (2004) Pollination effects of the sweat bee Lasioglossum vilosulum trichopse on genic-male lettuce, Appl. Entomo. Zool. 39(1) : 163-169, 2004.
**3.** Lindqvist, K. (1960) Inheritance studies in lettuce, Heriditas 46 :387-470.
**4.** Michelmore, R.W. et al. (1987) Genetic analysis of factors for résistance to downey mildew in lettuce, Plant Pathol., vol. 36, no4 : 499-514.
**5.** Michelmore, R.W. et al. (1993) Development of reliable PCR-based markers linked to downey mildew résistance genes in lettuce, Theor. Appl. Genet., vol. 85, n°8 : 985-993.
**6.** Ryder, E.J. (1963) An epistatically controlled pollen sterile in lettuce (Lactuca sativa L.), Proc. Am. Soc. Hort. Sci. 96 : 826-828
**7.** Ryder, E.J. (1967) A recessive male sterile gene in lettuce, Proc. Am. Soc. Hort. Sci 91 : 366-368
**8.** Ryder, E.J. (1971) Genetic Studies in Lettuce (Lactuca sativa L.), J.Amer.Soc.Hort.Sci 96(6) 826-828.
**9.** Ryder, E.J. (1979) Leafy Salad Vegetables, Avi. Pub. Co., page 30.
**10.** Maisonneuve, B. et al. (1994) Rapid mapping of two genes for résistance to downy mildew from Lactuca serriola to existing clusters of résistance genes, Theor. Appl. Genet. 89 : 96-104.
**11.** Kuang H., et al. (2004) Multiple genetic processes result in heterogeneous rates of evolution within the major cluster disease résistance genes in lettuce, The plant cell, Vol. 16, 2870-2894.
**12.** Kesseli R.V., et al. (1994) Analysis of a detailed linkage map of Lactuca sativa (lettuce) constructed from RFLP and RAPD markers, Genetics 136: 1435-1446.
**13.** Altman D.G., Bland J.M., 1994a. Statistics Notes: Diagnostic tests 1: sensitivity and specificity. British Medical Journal, 308:1552.
**14.** Altman D.G., Bland J.M., 1994b. Statistics Notes: Diagnostic tests 2: predictive values. British Medical Journal, 30:102.
**15.** Doyle J.J., and Doyle J.L., 1990. Isolation of plant DNA from fresh tissue. Focus 12,13-15.
**16.** Edwards K., Johnstone C. and Thompson C., 1991. A simple and rapid method for the preparation of plant genomic DNA for PCR analysis. Nucleic Acid Res 19:1349.
**17.** Michelmore R.W., Paran I., Kesseli R.V., 1991. Identification of markers linked to disease-resistance genes by bulked segregant analysis: a rapid method to detect markers in specific genomic regions by using segregating populations Proc Natl Acad Sci USA 88:9828-9832.
**18.** Paran I., Kesseli R., Michelmore R.W., 1991. Identification of restriction fragment length polymorphism and random amplified polymorphic DNA markers linked to downy mildew genes in lettuce, using near-isogenic lines Genome 34:1021-1027.
**19.** Stam P., & J.W. van Ooijen, 1996. JoinMap, version 2.0. Software for the calculation of genetic linkage maps. Ed. CPRO-DLO, 60 pp.
**20.** Tomas H.T., and Tanksley S.D., 1989. A rapid and inexpensive method for isolation of total DNA from dehydrated plant tissue. Plant Mol Biol Rep 12:106-109.
**21.** Welsh J, McClelland M., 1990. Fingerprinting genomes using PCR with arbitrary primers. Nucleic Acids Res, 18:7213-7218.
**22.** Williams J.K.G., Kubelik A.R., Livak K.J., Rafalsky J.A., Tynger S.V., 1990. DNA polymorphisms amplified by arbitrary primers are useful as genetic markers. Nucleic Acids Res, 18:6531-6535.
23. Williams J.K.G., Reiter R.S., Young R.M., Scolnik P.A., 1993. Genetic mapping of mutations using phenotypic pools and mapped RAPD markers. Nucleic Acids Res., 21(11):2697-2702.

## Revendications

1. Utilisation d'insectes de l'ordre des diptères à une concentration supérieure à 100 diptères par m² ou à 25 diptères par m³ pour transporter du pollen, dans un milieu fermé, de plantes *Lactuca sativa* mâles fertiles à des plantes *Lactuca sativa* mâles stériles.

2. Utilisation selon la revendication 1, où les plantes *Lactuca sativa* mâles stériles sont utilisées en tant que parents «femelles», et les plantes *Lactuca sativa* mâles fertiles sont utilisées en tant que parents «mâles», l'un des deux parents présentant comme caractéristique supplémentaire le fait d'être homozygote pour un gène lui conférant un phénotype détectable autre que la stérilité mâle, où ledit phénotype est un phénotype d'intérêt agricole, l'autre parent ne portant pas ce gène.

3. Utilisation selon la revendication 1 ou 2, où le transport du pollen permet la pollinisation des plantes *L. sativa* mâles stériles par les plantes *L. sativa* mâles fertiles.

4. Utilisation selon la revendication 3, où les plantes *Lactuca sativa* mâles stériles possèdent une stérilité mâle portée par un unique gène dominant, de préférence la stérilité mâle Ms7.

5. Graine de *Lactuca sativa* hybride, susceptible d'être obtenue par un procédé comprenant :
- une étape de culture dans un milieu fermé, de plantes *Lactuca sativa* phénotypiquement mâles stériles utilisées en tant que parents «femelles» et de plantes *Lactuca sativa* phénotypiquement mâles fertiles utilisées en tant que parents « mâles », à proximité les unes des autres, l'un des deux parents présentant comme caractéristique supplémentaire le fait d'être homozygote pour un gène lui conférant un phénotype détectable autre que la stérilité mâle, où ledit phénotype est un phénotype d'intérêt agricole, l'autre parent ne portant pas ce gène ;
- une étape de pollinisation par des diptères introduits dans le milieu fermé à l'époque de la floraison des plantes à une concentration supérieure à 100 diptères par m² ou à 25 diptères par m³, de préférence au moins 250 diptères par m² ou au moins 75 diptères par m³; et
- une étape de récolte des graines produites par les plantes mâles stériles.

6. Graine selon la revendication 5, où le second parent est également homozygote pour au moins un gène lui conférant un phénotype détectable autre que la stérilité mâle, non porté par l'autre parent.

7. Graine selon l'une quelconque des revendications 5 à 6, où la stérilité mâle des plantes utilisées en tant que parents femelles est d'origine monogénique, dominante et nucléaire.

8. Graine selon la revendication 7, où la stérilité mâle des plantes utilisées en tant que parents femelles est conférée par le gène Ms7.

9. Graine selon l'une quelconque des revendications 5 à 8, **caractérisée en ce qu'**elle
- présente un génotype mâle stérile, et
- est hétérozygote pour au moins un gène distinct conférant un phénotype détectable à la plante issue de cette graine, où ledit phénotype est un phénotype d'intérêt agricole.

10. Graine selon l'une quelconque des revendications 5 à 9, **caractérisée en ce que** son génome comporte une séquence ADN double brin de 650 à 700 nucléotides dont les extrémités 5' de chacun des deux brins débutent par la séquence TGCGTTCCAC (SEQ ID N°1).

11. Graine selon l'une quelconque des revendications 5 à 10, telle que ledit phénotype est conféré par un gène de résistance standard ou de résistance intermédiaire à une infection par un virus, une bactérie, un insecte ou un champignon.

12. Graine selon la revendication 11, telle que ladite graine présente un second gène de résistance standard ou intermédiaire distinct, sous forme hétérozygote.

13. Graine selon la revendication 11 ou 12, où le ou lesdits gènes de résistance standard ou intermédiaire sont choisis parmi les gènes suivants de résistance au brémia : Dm10, R17, Dm5/Dm8, R36/R37 (cluster 1), Dm1, Dm2, Dm3, Dm6, Dm14, Dm15, Dm16, Dm18 (cluster 2), ou Dm4, Dm7, Dm11, R38 (cluster 4) ; ou encore le gène Tu de résistance au TuMV (cluster 1) ; le gène Nr de résistance au Nasonovia (cluster 2) ; ou les gènes mol.1, mol.2 de résistance au LMV (cluster 4).


SEQUENCE LISTING

<110>  VILMORIN
       MICHEL, Herve
       SOUSSIN, Thierry

<120>  PRODUCTION DE GRAINES LACTUCA SATIVA HYBRIDES

<130>  B6739AGA

<140>  NOT YET KNOWN
<141>  2008-03-05

<150>  FR 07/01589
<151>  2007-03-05

<160>  6

<170>  PatentIn version 3.3

<210>  1
<211>  10
<212>  DNA
<213>  Artificial

<220>
<223>  OPBA05 primer

<400>  1
tgcgttccac                                                              10

<210>  2
<211>  675
<212>  DNA
<213>  Lactuca sativa

<400>  2
tgcgttccac caccgagact tcgacacctg gctgccccat tcgaactacg ccctgcgtag      60

agattcgtac gcccagcgta cgtgcgaggg tgcatcccta taaaaggcat gtgagacctt     120

cgggtttgtt gctcaatatt tcttctcttc tccccttatt ctttctattt cttggtaatt     180

tataccctcg aaaccccagt attattctcg agacctgaag caagtcccga agccctgaga     240

atcccgagaa gtaaagtttt cgagccgaaa ctctgcccgc gagaatcccg gttttcaaga     300

aagcgtatcg gtttcaccga agaatactac tcttagagtc gtagtgttgt tcgatcatct     360

tttgatcaag tgagtgtata ttccttttct tctaacacat agatacgaag tattctctac     420

aaaatacgtg ttatgtgttt gtatattatt tgcttatttg aaataattgt tgaatgaatg     480

atttgtacac gttctaagtt gtatataaat gtatatattt ttatctacta atatgttggg     540

tagaacatgg gtagaaagtt gttgtgagat gaaataaaat gatgagaggc ctcgatgttg     600

atgttgttaa tctagtcatc tagcggagta tggatgacga tcacggactc ttcctagact     660

gtccagtgga acgca                                                      675

<210>  3
<211>  24

<212>  DNA
<213>  Artificial

<220>
<223>  SCW09-A primer

<400>  3
gtgaccgagt agtcttaacc tagt                                              24


<210>  4
<211>  24
<212>  DNA
<213>  Artificial

<220>
<223>  SCW09-B primer

<400>  4
gtgaccgagt gtaacaacgt aaat                                              24


<210>  5
<211>  23
<212>  DNA
<213>  Artificial

<220>
<223>  B1F primer

<400>  5
gagaatagag tcttgtgatg gca                                               23


<210>  6
<211>  23
<212>  DNA
<213>  Artificial

<220>
<223>  B1R primer

<400>  6
cccgtaagac atggaagttc tct                                               23